# EUROPEAN PATENT APPLICATION

(11) **EP 4 850 681 A1**
(43) Date of publication of application: **09.12.2026**
(21) Application number: 25382567.3
(22) Date of filing: 03.06.2025
(51) Int. Cl.: A61K 38/17, A61P 25/28

(54) **E2F4 MUTANT FOR TREATMENT OF ALZHEIMER'S DISEASE**

(71) Applicant: Tetraneuron S.L, 28002 Madrid (ES); Consejo Superior De Investigaciones Científicas, 28006 Madrid (ES)
(72) Inventor: FRADE LÓPEZ, José María, E-28002 Madrid (ES); SÁNCHEZ-PUELLES PEÑARANDA, Cristina, E-28002 Madrid (ES); LÓPEZ SÁNCHEZ, Noelia, E-28002 Madrid (ES)
(74) Representative: ABG Intellectual Property Law, S.L.

(57) **Abstract**

The present invention relates to polynucleotides encoding for E2F4 mutants and their use and dosages in the treatment of Alzheimer's disease.

## Description

### FIELD OF INVENTION

The present invention is comprised within the field of biotechnology and biomedicine, specifically in methods for the treatment of Alzheimer's disease and dosages of medicine for its treatment.

### BACKGROUND ART

Alzheimer's disease (AD) is an irreversible neurodegenerative disease leading to dementia. This disease, which is defined by memory loss and difficulties in language, problem solving and other cognitive factors, affects 50 million individuals globally. Synaptic failure is one of the first alterations that can be observed in AD, preceding neuronal death, and both processes are proposed as etiological factors for AD. In addition, AD is characterized by the aggregation in the extracellular space of β-amyloid (Aβ) peptide, which forms senile plaques, and the presence of intracellular deposits of hyperphosphorylated tau protein, forming neurofibrillary tangles. Activation of the immune system and neuroinflammation are also hallmarks of AD.

Multifactorial gene therapy based on a modified form of E2F4 unable to become phosphorylated at a conserved Thr motif (Thr248/Thr250 in human E2F4 and Thr249/Thr251 in mouse E2F4), which we have referred to as E2F4DN, has been suggested for preventing or delaying the onset of AD, but not for the treatment of the disease once it has developed. Despite many decades of work, we still lack effective therapies against this stage of the disease, probably due to its complex etiology, which requires a multifactorial therapeutic approach addressing all these etiological factors.

### SUMMARY OF THE INVENTION

The inventors have investigated the role of a dominant negative form of human E2F transcription factor 4 (hE2F4DN) in synaptic failure and neuronal death, as well as its cognitive consequences, by administering an AAV vector capable of inducing hE2F4DN expression specifically in neurons. They found an improvement in memory in homozygous 5xFAD mice (h5xFAD) mice, which was recovered when hE2F4DN was expressed in mice that already show absence of memory formation. hE2F4DN was also able to recover synapse function and plasticity, and prevent neuronal death in these mice. This demonstration that hE2F4DN is involved in the recovery of synaptic plasticity is of vital importance when referring to AD, since the loss of synaptic plasticity begins long before symptoms become apparent.

A first aspect of the invention provides a polynucleotide encoding a mutant form of the human E2F4 transcription factor in which positions 248 and/or 250 are mutated so that they cannot be phosphorylated for use in the treatment of Alzheimer's Disease in a patient diagnosed as having Alzheimer's Disease and having mild, moderate or severe dementia.

Another aspect provides a pharmaceutical composition comprising an adeno-associated virus serotype 9 (AAV9) vector which comprises a polynucleotide that encodes a mutant form of the human E2F4 transcription factor in which positions 248 and/or 250 are mutated so that they cannot be phosphorylated for use in the prevention and/or treatment of Alzheimer's disease wherein the pharmaceutical composition is administered to achieve between 0.5 × 10¹⁴ and 6 × 10¹⁴ viral genomes (vg) of the adeno-associated virus serotype 9 (AAV9) vector per kg of brain.

### BRIEF DESCRIPTION OF THE FIGURES

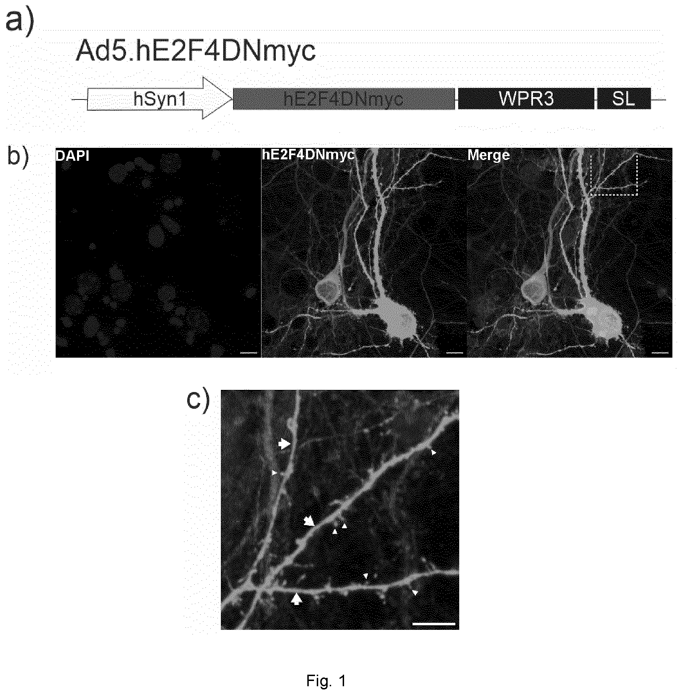

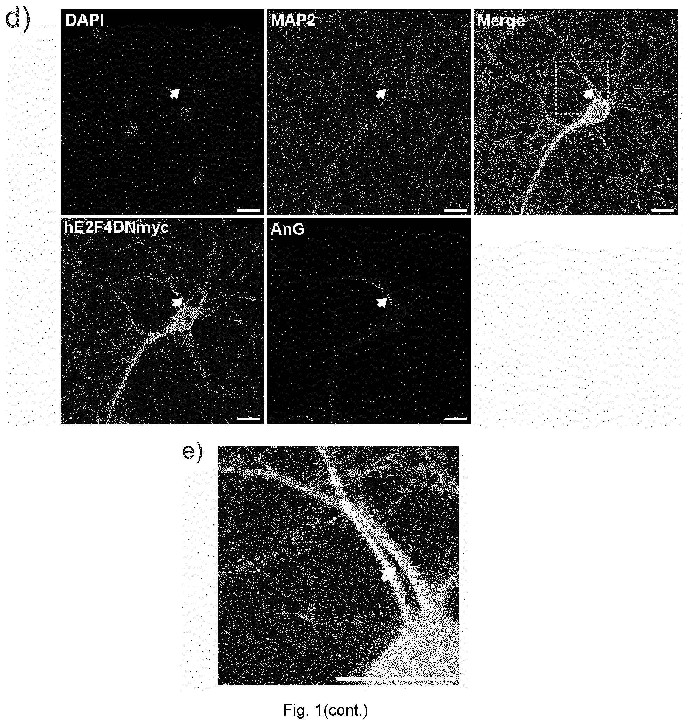

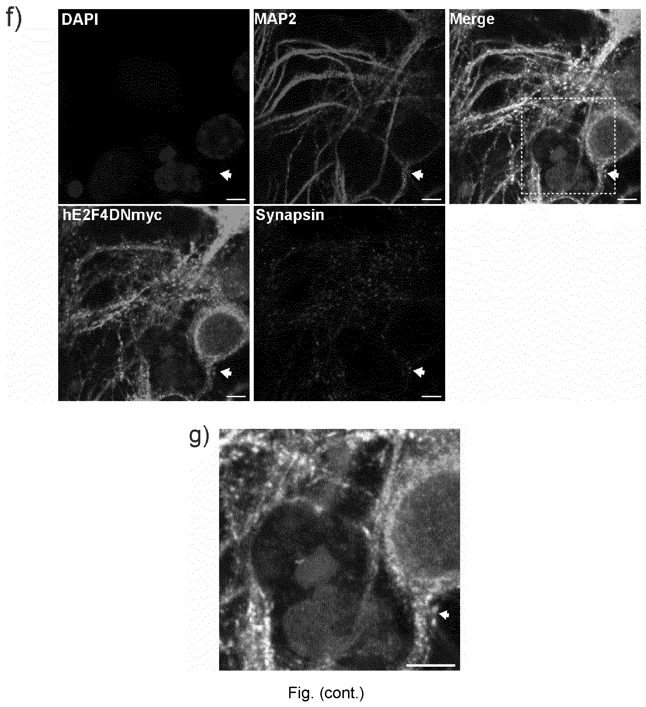

**Figure 1****: hE2F4DN expression detected throughout the neuron, including dendritic spines and axons.** a) Schematic diagrams showing the genomic sequence of Ad5. hSyn1-hE2F4myc-WPR3SL (Ad5.hE2F4DNmyc) vector. b) Projection of confocal planes showing hE2F4DNmyc expression in dendrites and dendritic spines. c) High magnification of the white square in panel b illustrating dendrites (arrows) with spines (arrowheads). d) Projection of confocal planes showing hE2F4DNmyc expression in axons of hippocampal neurons. Dendrites are revealed as MAP2+ processes. The axon initial segment (arrow) expressing Ankyrin G (AnG) identifies the proximal part of the axon. e) High magnification of the white square in panel d illustrating axon initial segment (arrow) expressing AnkyrinG and colocalizing with hE2F4DNmyc. f) Projection of confocal planes showing hE2F4DNmyc expression in synapses. Notice proximal localization and colocalization between hE2F4DNmyc and synapsin (arrow) in merge images. g) High magnification of the white square in panel e illustrating spines (arrow) with proximal localization of synapsin and hE2F4DNmyc expression. Scale bar: 10 µm (g), 5 µm (b, e, f), 20 µm (c, d).

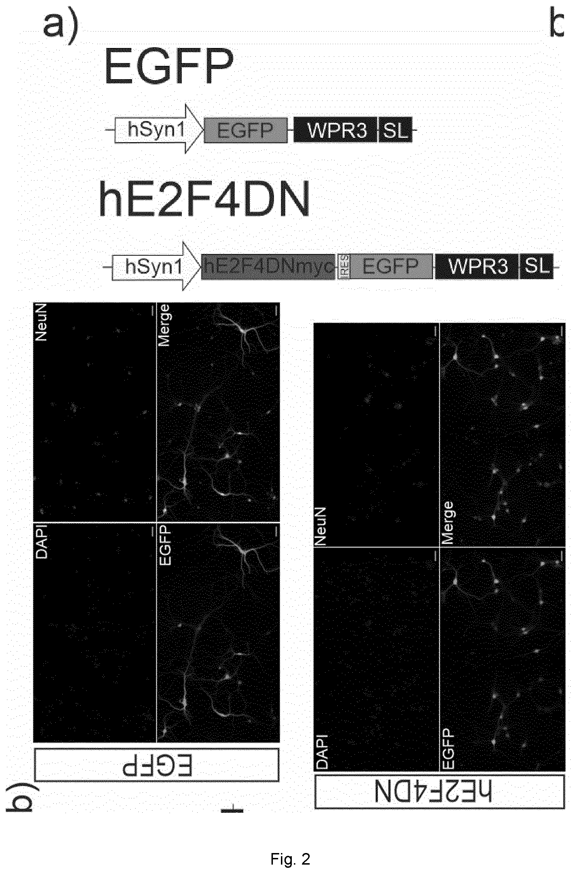

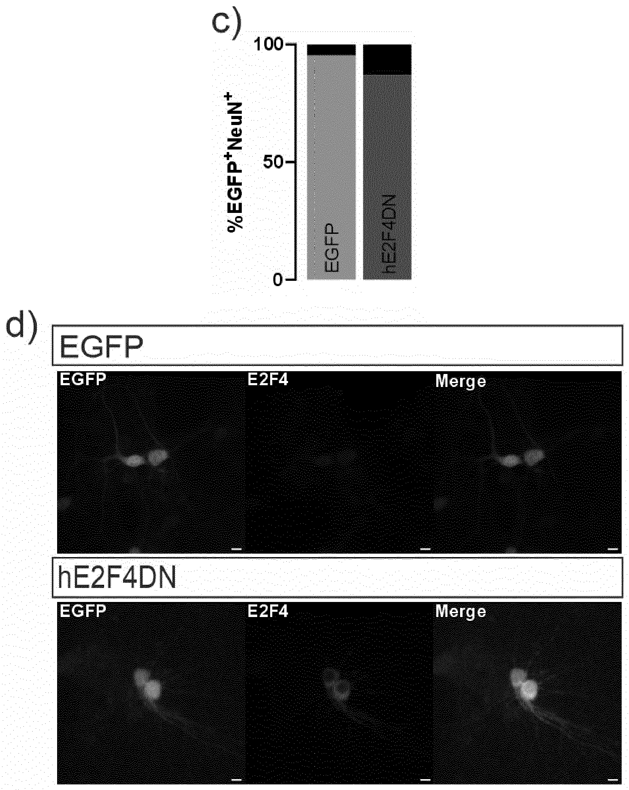

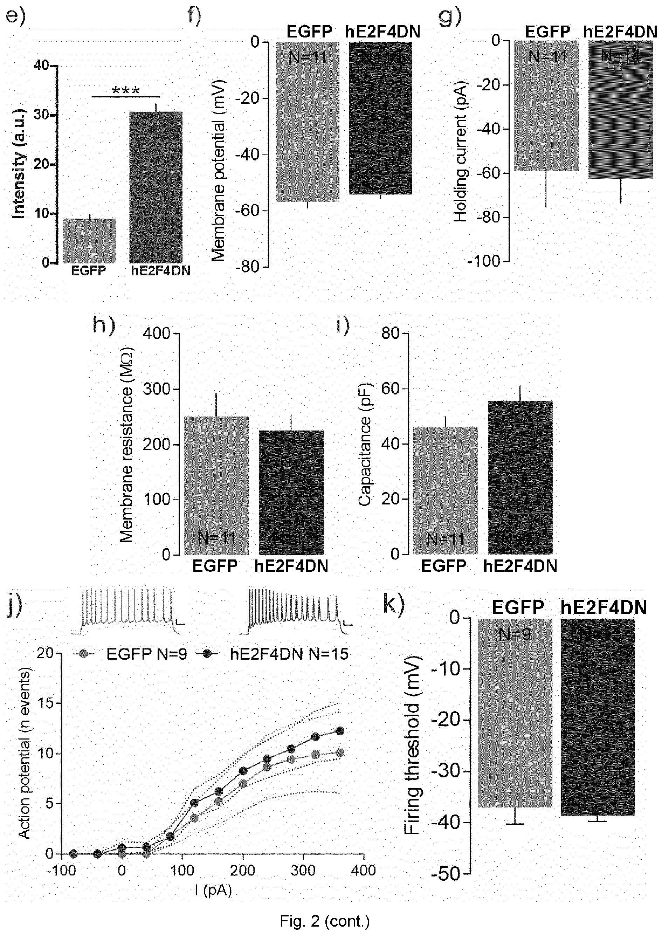

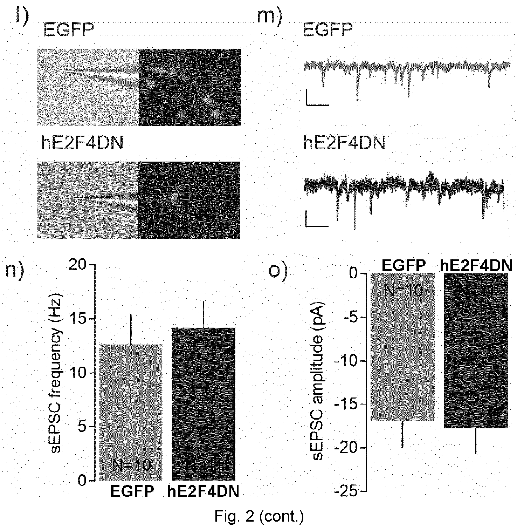

**Figure 2****. Neuronal expression of hE2F4DNmyc does not affect basal membrane properties, and neuron excitability and spontaneous activity.** a) Schematic diagrams showing the genomic sequences of Ad5.hSyn1-EGFP-WPR3SL (EGFP) and Ad5. hSyn1-hE2F4myc-IRES-EGFP-WPR3SL (hE2F4DN) vectors. b) Representative microscopy images of primary cultured neurons transduced with either Ad5.hSyn1-EGFP-WPR3SL (EGFP) or Ad5. hSyn1-hE2F4myc-IRES-EGFP-WPR3SL (hE2F4DN) vectors. Nuclei labeled with DAPI, neurons labeled with the neuronal marker NeuN and vector expression is detected with EGFP (THUNDER fluorescence microscope). Neuronal selectively is defined as the co-expression of the neuronal marker NeuN and EGFP. Scale bar: 50 µm. c) Quantifications of transduction efficiency are shown for EGFP and hE2F4DN, quantified as the percentage NeuN cells expressing EGFP (654 and 1012 neurons were counted for EGFP and hE2F4DN, respectively). d) Representative images showing E2F4 expression in neurons transduced with EGFP, top, and hE2F4DN, bottom. (epifluorescence microscope). Scale bar: 10 µm. e) Quantification of the intensity of E2F4 in transduced neurons, detected as EGFP-positive cells. E2F4 intensity is represented as mean ± s.e.m. Quantification from 51 neurons transduced with EGFP and 34 neurons transduced with hE2F4DN, from 3 independent experiments. ****p<* 0.001, (unpaired Student's *t* test). f) Resting membrane potential. g) Holding current. h) Membrane resistance. i) Capacitance. j) Upper traces, representative firing profiles of neurons transduced for 3 days with EGFP or hE2F4DN, scale bar: 10mV, 100 ms. Bottom, number of action potentials at different current pulses. k) Firing threshold. I) Bright field (right) and fluorescence (left) representative images of patch-clamped primary hippocampal neurons expressing EGFP (top) or hE2F4DN (bottom). m) Representative sEPSCs traces for each condition, as indicated. Scale bar: 10 pA, 50 ms. h) Frequency of sEPSCs. i) Amplitude of sEPSCs. f, g, h, i, j, k, n, o) Graphs represent mean ± s.e.m. of neurons expressing either EGFP or hE2F4DN. The number of cells analyzed (from 3 independent experiments) is indicated on each graph.

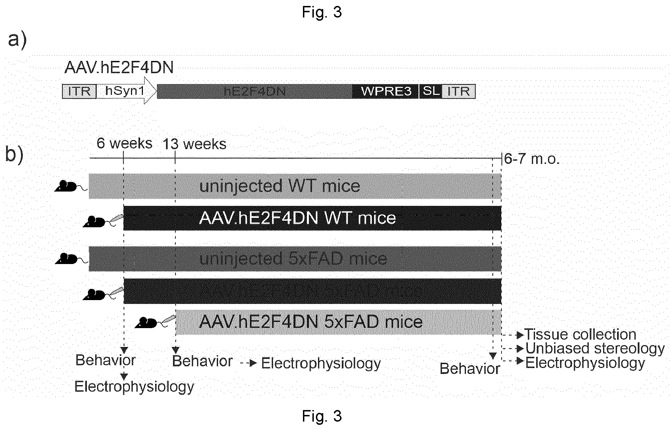

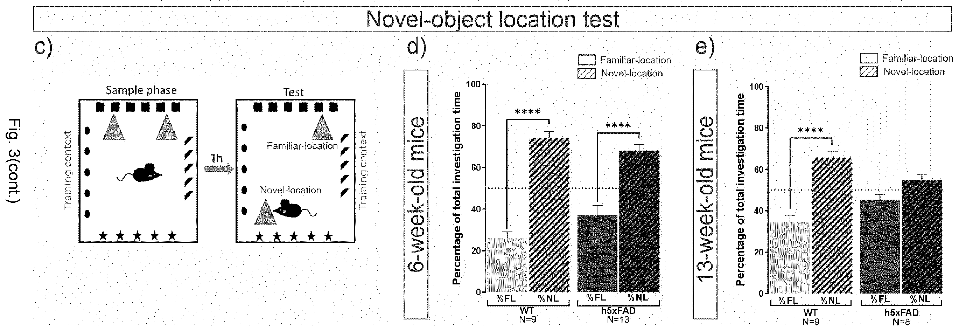

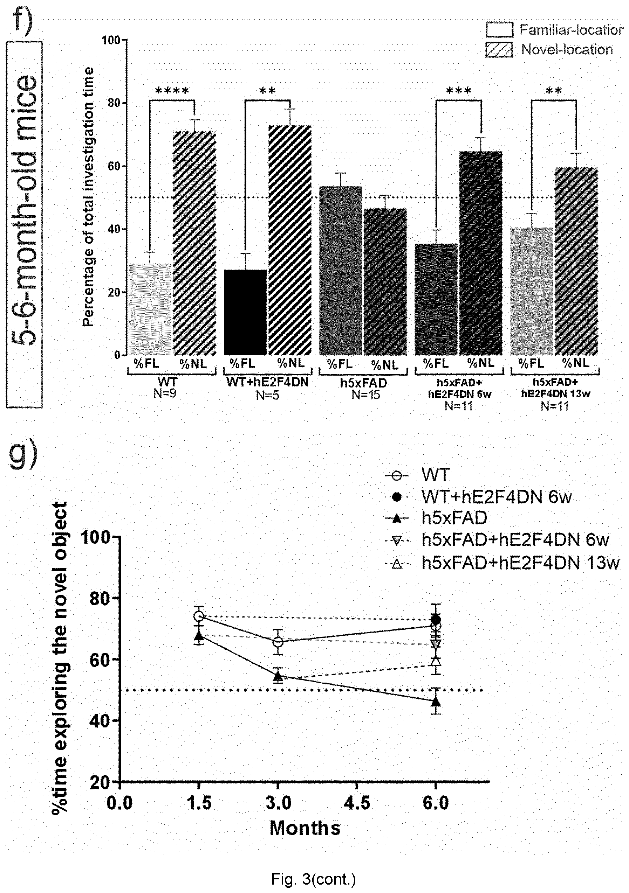

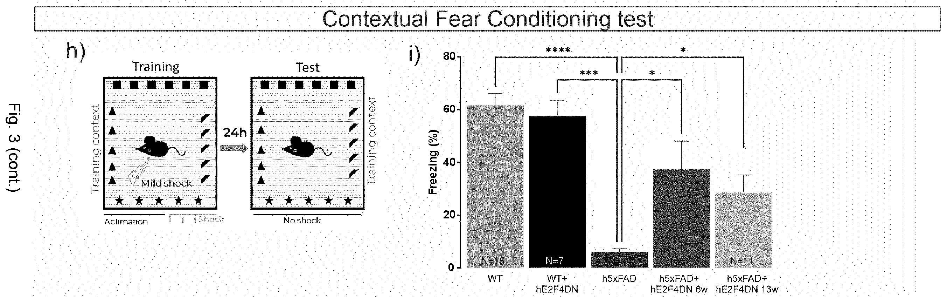

**Figure 3****. Intravenous injection of AAV.hE2F4DN in h5xFAD mice recovers hippocampal-dependent memory.** a) Schematic diagrams showing the genomic sequence of AAV-AS.hSyn1-hE2F4-WPR3SL (AAV.hE2F4DN) vector. b) Schematic representation of the design of the experimental groups and the timeline for treatments and experiments. Hippocampal-dependent memory was tested in the NOL and CFC tasks at 5-6 months of age in WT mice (control and injected at 6 weeks with AAV. hE2F4DN) and h5xFAD mice (control and injected with AAV.hE2F4DN at 6 and 13 weeks). c) Schematic draw of the novel-object location task. (d-f) Bar graph representing the percentage of investigation time of the novel (%NL) and familiar (%FL) located object in the NOL test at 6 weeks (d), 13 weeks (e) and 5-6 months old (f). g) Timeline of the percentage of investigation of the NL in the NLO test at different ages. h) Schematic draw of CFC task. i) Percentage of freezing to the context during the 5 minutes of the CFC test. All graphs represent mean ± s.e.m. The number of mice analyzed is indicated on each graph. **** *p*< *0.0001***p<* 0.001, ***p<0.01,* **p*<0.05 (Mann Whitney test for %NL vs. %FL comparison; Kruskal-Wallis test followed by Dunn's multiple comparisons test for CFC).

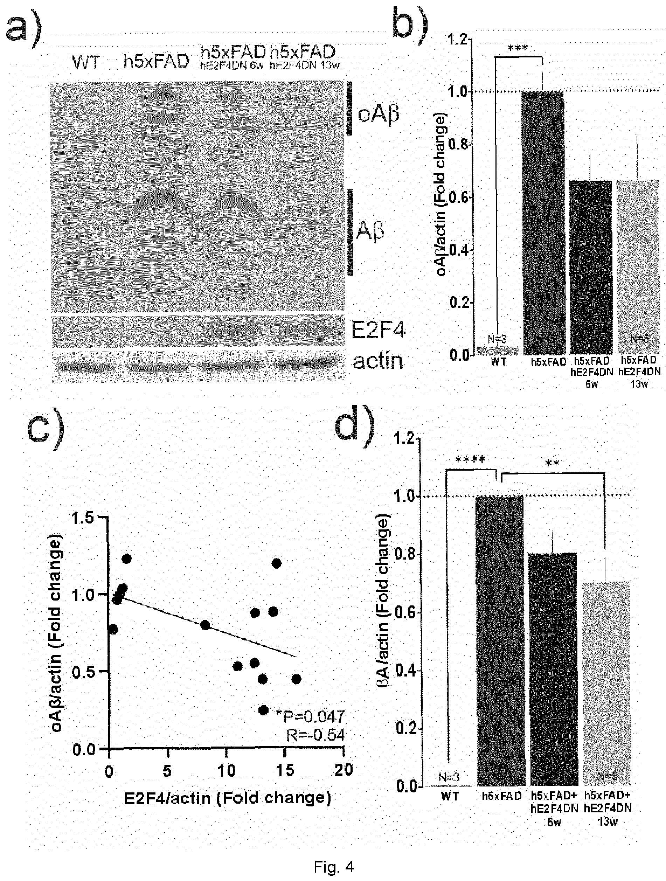

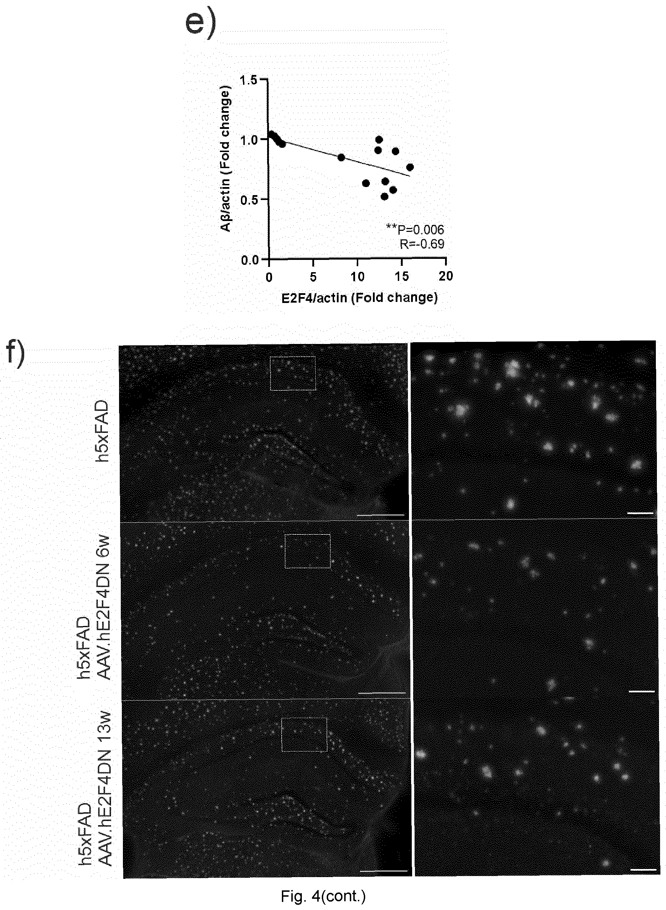

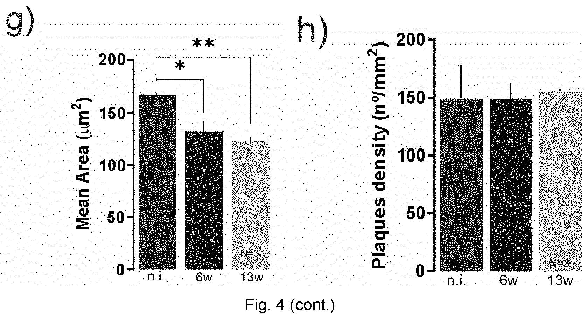

**Figure 4****. hE2F4DN neuronal expression reduces Aβ pathology in h5xFAD mice.** (a-e) Quantification of soluble Aβ levels in hippocampal homogenates of WT and h5xFAD mice, with or without treatment with AAV.hE2F4DN, administrated at 6 o 13 weeks of age. a) Representatives immunoblots of Aβ species, E2F4 and actin. b) Bar graph representing oligomeric Aβ (oAβ) levels. c) Correlation between oAβ and E2F4 levels (**p* < 0.05; Pearson's *R* correlation test). d) Bar graph representing monomeric Aβ levels. e) Correlation between monomeric Aβ and E2F4 levels (***p* < 0.01; Pearson's *R* correlation test). Data were normalized for the non-treated h5xFAD group. b, d) The number of mice analyzed is indicated on each graph. Individual replicates are shown with mean ± SEM (bars and error bars) overlaid (one-way ANOVA, followed by Dunnett's multiple comparisons test). (f-h) Quantification of Aβ plaques in hippocampal slices of h5xFAD mice, with or without treatment with AAV.hE2F4DN, administrated at 6 o 13 weeks of age. f) Left, representative microscopy images of Aβ plaques in the hippocampus of 6-month-old h5xFAD mice, untreated or administered with AAV.hE2F4DN at 6 and 13 weeks of age. Scale bar: 500 µm. No plaques were observed in WT. Right, insets showing a section of the CA1 region in higher magnification, corresponding to squares in the hippocampal left images. Scale bar: 50 µm. g) Bar graph showing the mean area of the plaques from the different groups. * *p<* 0.05***p*< 0.01 (One-way ANOVA test followed by Dunnett's multiple comparisons test). h) Bar graph showing plaque density quantification. All graphs represent mean ± s.e.m. The number of mice analyzed is indicated on each graph.

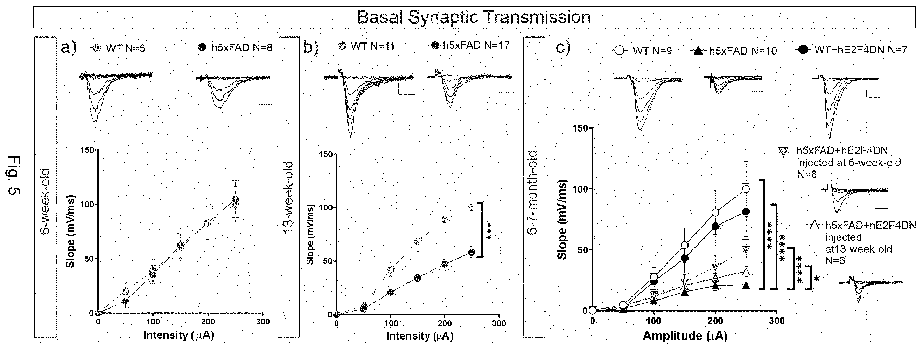

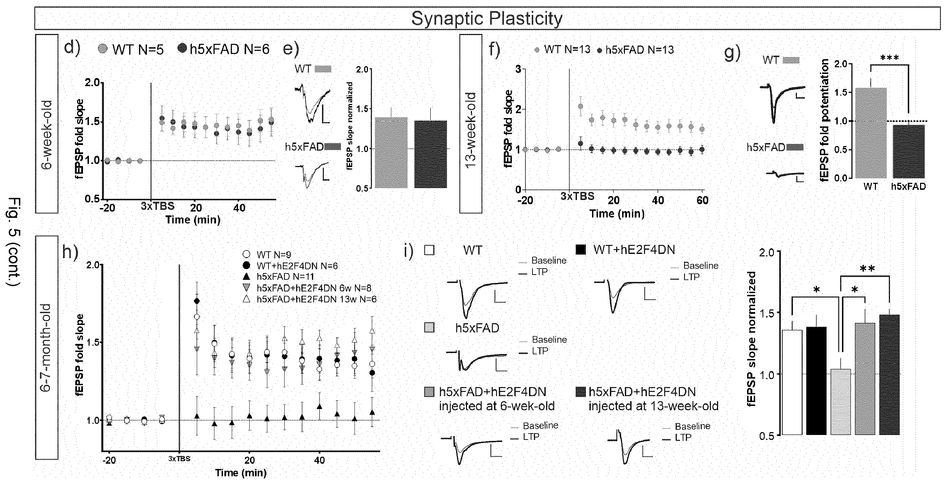

**Figure 5****. Intravenous injection of AAV.hE2F4DN in h5xFAD mice rescues LTP.** (a-c) Input-output curves of the fEPSPs evoked by stimulation of Schaffer collateral fibers and recorded in the hippocampal CA1 region of 6-weeks-old WT and h5xFAD mice (a), 13-weeks-old WT and h5xFAD mice (b) and 6-7 months of age WT mice (control and injected at 6 weeks with AAV. hE2F4DN) and h5xFAD mice (control and injected with AAV.hE2F4DN at 6 and 13 weeks). (c). Top: Representative traces of fEPSPs superimposed at 10, 50, 100, 150, 200 and 250 µA of stimulation intensity. Bottom: Input-output curve, *p* values were determined with a two-way ANOVA, followed by Bonferroni post hoc test. N represents the number of animals analyzed. (d-i) LTP recordings, induced in the CA1 in hippocampal coronal slices taken from WT or h5xFAD mice (3xTBS) stimulating axons arising from the CA3 in 6-weeks (d), 13-weeks (f) and 6-7 months of age (h). (e, g, i) Left, representative traces of fEPSPs of the basal response (thin line) and after LTP induction (thick line). Right, quantification of mean fEPSPs slopes 45 min after induction at 6 weeks (e), 13-weeks (g) and 6-7 months of age (i). (g)****p*<0.001 (determined with a Mann-Whitney test). (i)* *p*<0.05, **p<0.01 (Kruskal-Wallis followed test Dunn's multiple comparisons test). N represents the number of animals. All graphs represent mean ± s.e.m. Scale bars represent 0.5 mV, 10 ms.

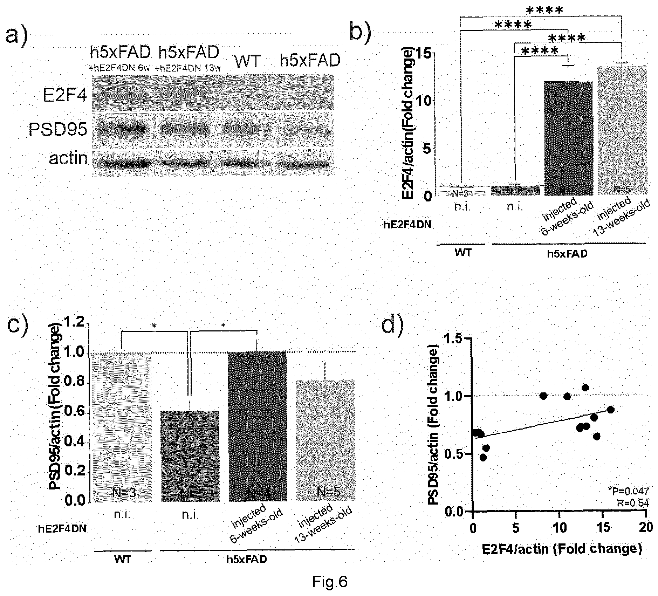

**Figure 6****. *In vivo* administration of AAV.hE2F4DN increases PSD-95 levels in h5xFAD mice.** (a-d) PSD-95 protein levels measured in hippocampal homogenates from 6-months-old WT and h5xFAD mice, with or without treatment, administrated at 6 o 13 weeks of age. a) Representative immunoblot of PSD-95, E2F4 and actin. b) Bar graph representing E2F4 levels, confirming the expression after AAV.hE2F4DN administration. Data were normalized for the non-treated h5xFAD group. ****p<0.0001 (One-way ANOVA, Tukey's multiple comparisons test). c) Bar graph representing PSD-95 levels. Data were normalized for the WT group. *p<0.5 (One-way ANOVA, Dunnett's multiple comparisons test). d) Correlation between PSD-95 and E2F4 levels (**p* < 0.05; Pearson's *R* correlation test). Bar graphs represent mean ± s.e.m.

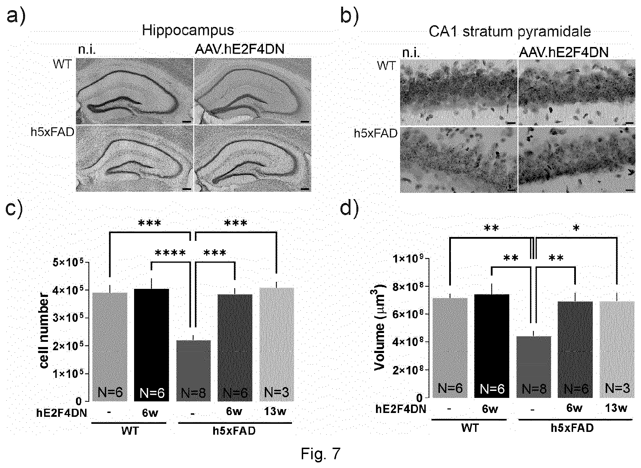

**Figure 7****. Intravenous injection of AAV.hE2F4DN in h5xFAD mice improves neuronal survival.** a) Nissl-stained hippocampal sections from WT (top) and h5xFAD (bottom) mouse uninjected (left) and injected with AAV.hE2F4DN (right). Images obtained on Leica Thunder Imager DM i8 brightfield microscope with a 20× objective and tile scanned. Scale bar: 200 µm. b) Representative images of Nissl-stained CA1 stratum pyramidale from a WT mouse (top) and homozygous 5xFAD mice (bottom) either uninjected (left) or injected with AAV.hE2F4DN (right). Images obtained on Leica Thunder Imager DM i8 microscope (63x objective). Scale bar: 10 µm. c) Bar graph showing number of neurons in CA1 in 6-month-old mice: WT, WT injected at 6 weeks with AAV.hE2F4DN, h5xFAD and h5xFAD injected with AAV.hE2F4DN at 6 weeks. d) Bar graph showing CA1 volume of 6-month-old mice: WT, control injected at 6 weeks with AAV.hE2F4DN, h5xFAD and h5xFAD injected with AAV.hE2F4DN at 6 weeks. Numbers inserted in the bars represent the number of mice used for the analysis. **** *p<* 0.0001, ****p<* 0.001, ***p*<0.01*,* **p*<0.05 (One-way ANOVA test followed by a Dunnett's multiple comparisons test). Graphs represent mean ± s.e.m.

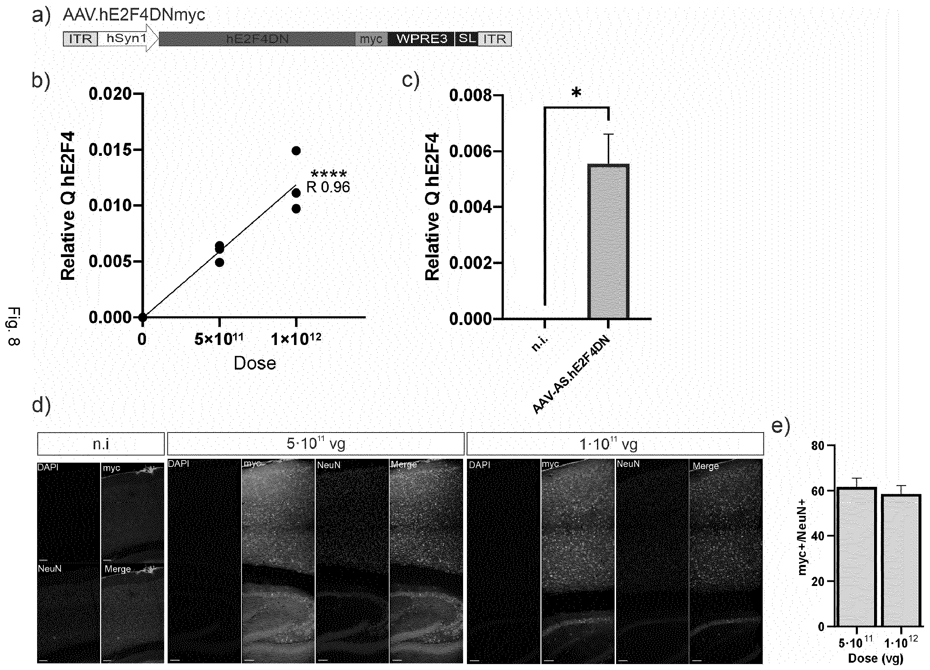

**Figure 8****. AAV-AS vector characterization.** a) Schematic diagrams showing the genomic sequence of AAV-AS.hSyn1-hE2F4myc-WPR3SL (AAV.hE2F4DNmyc) vector. b) Ratio between *hE2f4dn* and *Rps18* gene expression, quantified by qRT-PCR from cDNA obtained from the cerebral cortex of WT mice injected at 6 weeks of age with the AAV.hE2F4DNmyc vector and sacrificed one month later. (*****p* <0.0001; Pearson's *R* correlation test). c) Ratio between hE2f4dn and Rps18 gene expression, quantified by qRT-PCR from cDNA obtained from the cerebral cortex of h5xFAD mice injected at 6 weeks of age with the AAV.hE2F4DNmyc vector (5×10¹¹vg) and sacrificed at 7 months of age. d) Representative images of the cerebral cortex and hippocampus from WT mice administered at 6 weeks of age with the AAV.hE2F4DNmyc vector at the doses indicated and sacrificed 1 month later. Scale bar: 100 µm. e) Percentage of cortical neurons, labeled with NeuN, expressing hE2F4DNmyc 1 month after delivery of the AAV.hE2F4DNmyc vector at the indicated doses.

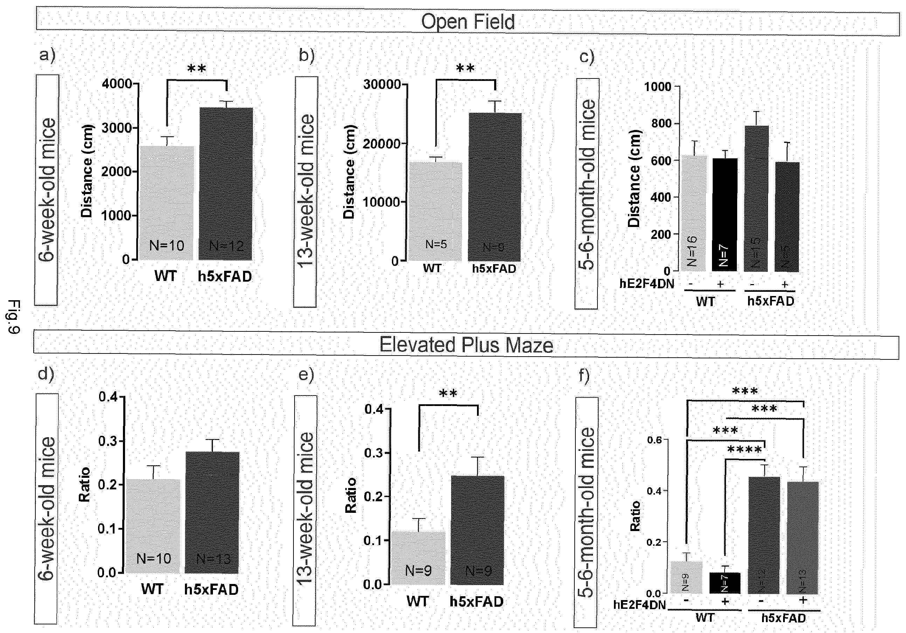

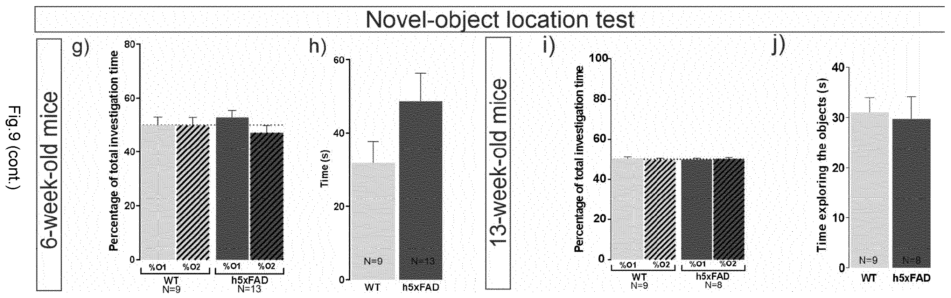

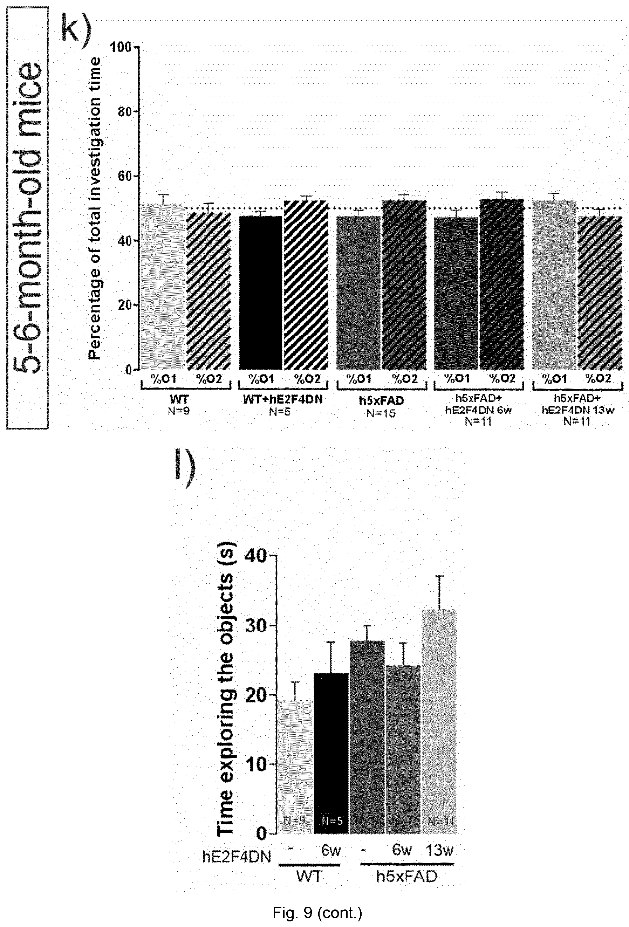

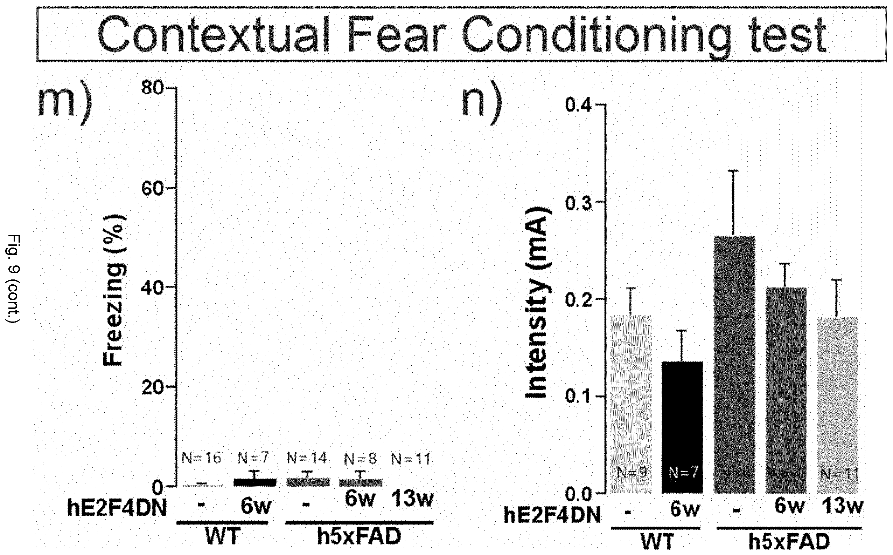

**Figure 9****. Behavioral characterization of intravenous injected h5xFAD mice with AAV.hE2F4DN. .** (a-f) Behavioral characterization of 6-weeks-old WT and h5xFAD mice, 13-weeks-old WT and h5xFAD mice and WT mice (control and injected at 6 weeks with AAV. hE2F4DN) and h5xFAD mice (control and injected with AAV.hE2F4DN at 6 and 13 weeks) at 5-6 months of age.Open Field test in 6-weeks-old (a, ***p*< 0.01 t-test), 13-weeks-old (b***p*< 0.01 t-test), and 5-6 months old (c) mice. EPM in 6-weeks-old (d), 13-weeks-old (e, ***p*< 0.01 t-test) and 5-6 months old (f, ****p*< 0.001, *****p*<0.0001, One-way ANOVA test followed by a Tukey's HSD post hoc test) mice. (g-l) NOL in 6-weeks-old WT and h5xFAD mice, 13-weeks-old WT and h5xFAD mice and 5-6 months-old WT mice (control and injected at 6 weeks with AAV. hE2F4DN) and h5xFAD mice (control and injected with AAV.hE2F4DN at 6 and 13 weeks). Percentage of exploration of the objects in the sample phase in 6-weeks-old (g), 13-weeks-old (i) and 5-6 months old (k) mice. Time exploring both objects in the choice phase in 6-weeks-old (h), 13-weeks-old (j) and 5-6 months old (l) mice. (m-n) CFC in WT mice (control and injected at 6 weeks with AAV. hE2F4DN) and h5xFAD mice (control and injected with AAV.hE2F4DN at 6 and 13 weeks) at 5-6 months of age. m) Percentage of freezing displayed by WT mice (control and injected at 6 weeks with AAV.hE2F4DN) and h5xFAD mice (control and injected with AAV.hE2F4DN at 6 and 13 weeks of age) during the first 3 minutes of CFC training, before shock exposure. n) Pain threshold evaluated as the intensity in which the animals vocalized or jumped. All graphs represent mean ± s.e.m. The number of mice analyzed is indicated on each graph.

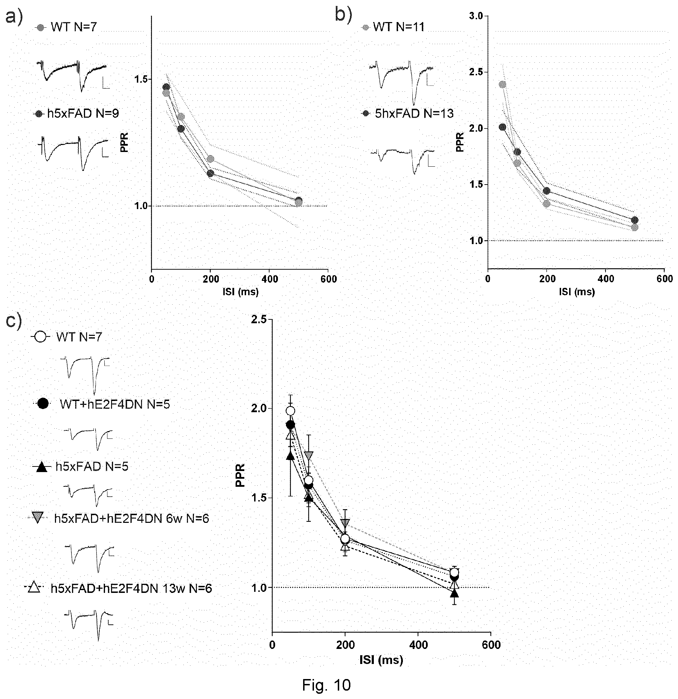

**Figure 10****. Intravenous injection of AAV.hE2F4DN in h5xFAD mice does not alter presynaptic function.** Paired-pulse facilitation (PPF) in hippocampal slices from 6-weeks-old WT and h5xFAD mice (a), 13-weeks-old WT and h5xFAD mice (b) and WT mice (control and injected at 6 weeks with AAV. hE2F4DN) and h5xFAD mice (control and injected with AAV.hE2F4DN at 6 and 13 weeks) at 6-7 months of age. a-c) Left: representative traces of the fEPSPs obtained with an ISI of 50 ms. Right: PPFs (i.e. fEPSP values obtained in the second response divided by the fEPSP values obtained in the first response) plotted against the interstimulus intervals (ISIs) as a measure of PPF. N represents the number of animals analyzed.

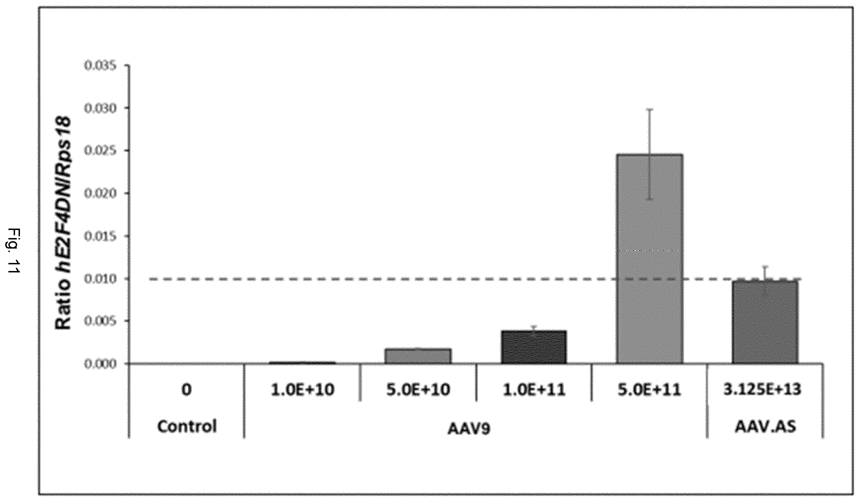

**Figure 11****.** Comparison of hE2F4DN/Rps18 ratio in the cerebral cortex of mice injected intravenously or intracisternally with AAV.hE2F4DN or different doses of AAV9-hE2F4DN, respectively.

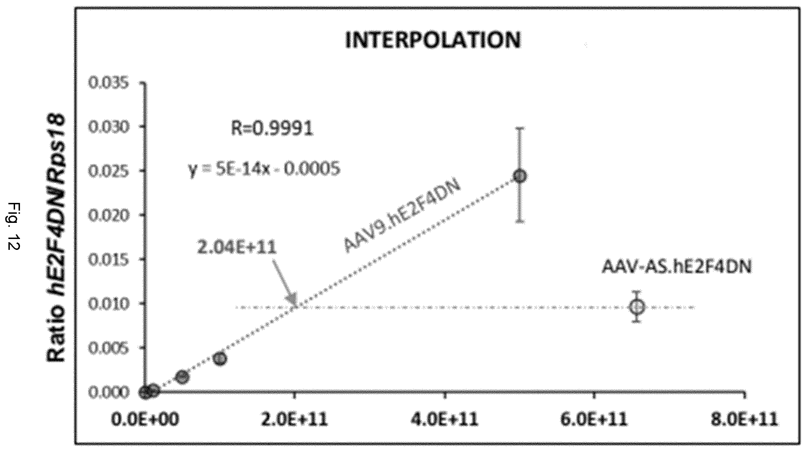

**Figure 12****.** Interpolation of the hE2F4DN/Rps18 ratio in the cerebral cortex of mice injected with AAV9-hE2F4DN with the effective dose of AAV.hE2F4DN. This interpolation indicated that the therapeutic dose of AAV9-hE2F4DN was 2.04 × 10¹¹ total vg/brain.

### DETAILED DESCRIPTION OF THE INVENTION

As previously stated, the inventors have found that by administering an AAV vector capable of inducing hE2F4DN expression specifically in neurons they obtained an improvement in memory in h5xFAD mice, which was recovered when hE2F4DN was expressed in mice that already showed an absence of memory formation. Remarkably, when hE2F4DN treatment was applied where synaptic and memory deficits were already present in h5xFAD mice, at 3 months of age, full recovery of long-term potentiation (LTP) was observed along with decreased levels of β-amyloid and increased levels of the postsynaptic marker PSD-95. These results indicate the potential of hE2F4DN treatment to reverse established pathological and functional impairments associated with Alzheimer's disease. The observed reduction in neurotoxic β-amyloid levels, increase in pro-synaptic PSD-95 levels, and restoration of LTP demonstrate a therapeutic utility for hE2F4DN in improving synaptic integrity and cognitive function. This thereby indicates its potential as a disease-modifying treatment for Alzheimer's disease, particularly when symptoms are already present.

### Medical use of the invention

Hence, a first aspect of the invention provides a polynucleotide encoding a mutant form of the human E2F4 transcription factor in which positions 248 and/or 250 are mutated so that they cannot be phosphorylated for use in the treatment of Alzheimer's Disease in a patient diagnosed as having Alzheimer's Disease and having mild, moderate or severe dementia, from here onwards the medical use of the invention.

The term "polynucleotide" as used herein refers to any polyribonucleotide or polydeoxyribonucleotide, which may be unmodified RNA or DNA or modified RNA or DNA. Thus, for example, polynucleotides as defined herein include but are not limited to single-stranded and double-stranded DNA. The RNA of the region, a hybrid molecule containing DNA and RNA, may be single-stranded, or more typically double-stranded, or contain single-stranded and double-stranded regions. The term "polynucleotide" specifically includes cDNA. The term includes DNA (including cDNA) and RNA containing one or more modified bases. As such, DNA or RNA whose backbone is modified for stability or other reasons is also the intention of the term "polynucleotide" herein. In addition, DNA or RNA containing rare bases such as inosine or modified bases such as tritiated bases are also included in the term "polynucleotide" as defined herein. In general, the term "polynucleotide" encompasses all chemically, enzymatically, and / or metabolically modified forms of unmodified polynucleotides, as well as the chemical forms of DNA and RNA that are characteristic of viruses and cells, including simple and complex cells. The term "polynucleotide" as used herein also refers to a polynucleotide which encodes a mutant form of the human E2F4 transcription factor.

The term "human E2F4 transcription factor" or "E2F4" as used herein, refers to a protein member of the E2F transcription factor family, which plays a crucial role in the control of cell cycle and in pathways related to tumor suppression . It is also a target of the transforming proteins of small DNA tumor viruses. The "human E2F4 transcription factor" protein refers to the protein identified in the Uniprot database as Q16254 entry version 229 of the 23 April 2025. The human E24F transcription factor according to the present invention is a "mutant form", i.e., it refers to a protein which has been altered in relation to the wild-type protein by at least one or more changes in its amino acid sequence, such as deletion, insertion, substitution, inversion. In the present context, the mutant form of the human E2F4 transcription factor" is mutated in positions 248 and/or 250 so that they cannot be phosphorylated. In a particular embodiment of the medical use of the invention, the position 248 of the mutant form of the human E2F4 transcription factor is mutated so that it cannot be phosphorylated. In another particular embodiment of the medical use of the invention, the position 250 of the mutant form of the human E2F4 transcription factor is mutated so that it cannot be phosphorylated. In yet another particular embodiment of the medical use of the invention, the position 248 and the position 250 of the mutant form of the human E2F4 transcription factor are mutated so that they cannot be phosphorylated.

The terms "position 248" or "position 250" as used herein refers to the position of the amino acid within the amino acid sequence of the protein, i.e., starting the count from the first amino acid at the N-terminus of the protein, and carrying on until reaching the 248^{th} or, 250^{th} or n^{th} position in the amino acid sequence. The numbering can be counted ignoring the first methionine of the amino acid sequence. In that case as an example and in the present context, the positionally equivalent to the position 248 would be 247. Therefore, in a particular embodiment of the medical use of the invention, the position 248 and/or position 250 of the mutant form of the human E2F4 transcription factor refers to a positionally equivalent amino acid. As it is used herein, the term "positionally equivalent" refers to the position of an amino acid of an E2F4 protein which, by means of multiple amino acid sequence alignment of E2F4 protein, corresponds to positions 248 and/or 250 of the human E2F4 protein.

The term "phosphorylated" or "phosphorylation" as used herein, refers to the protein reversible post-translational modification in which an amino acid residue is phosphorylated by a protein kinase by the addition of a covalently bound phosphate group. Phosphorylation alters the structural conformation of a protein, causing it to become activated, deactivated, or otherwise modifying its function. The reverse reaction of phosphorylation is called dephosphorylation and is catalyzed by protein phosphatases. The amino acids most commonly phosphorylated are serine (Ser o S), threonine (Thr o T), tyrosine (Tyr o Y), and histidine (His o H), being that the indication of an amino acid being phosphorylated is normally the addition of the "p" before the amino acid letter, i.e., pS or pT refer to phosphorylated serine or phosphorylated threonine. In a particular embodiment of the medical use of the invention, the position 248 and/or position 250 of the human E2F4 transcription corresponds to a Thr (T248 and/or T250).

While some amino acids are amenable to being phosphorylated others such as alanine (Ala o A), glutamine (Gln o Q), phenylalanine (Phe or F), and valine (Val or V), are not, partly due to the lack of a hydroxyl group as an acceptor of the phosphate group. In a particular embodiment of the medical use of the invention, the position 248 of the mutant form of the human E2F4 transcription factor is mutated to an alanine (T248A), glutamine (T248Q), phenylalanine (T248F), leucine (T248L), Isoleucine (T248I), methionine (T248M), glycine (T248G), phenylalanine (T248F), tryphophan (T248W), proline (T248P) or valine (T248V), preferably an alanine (T248A). In another particular embodiment of the medical use of the invention, the position 250 of the mutant form of the human E2F4 transcription factor is mutated to an alanine (T250A), glutamine (T250Q), phenylalanine (T250F), leucine (T250L), Isoleucine (T250I), methionine (T250M), glycine (T250G), phenylalanine (T250F), tryphophan (T250W), proline (T250P) or valine (T250V), preferably an alanine (T250A). In yet another particular embodiment of the medical use of the invention, the position 248 and the position 250 of the mutant form of the human E2F4 transcription factor are mutated to an alanine (T248A and T250A), glutamine (T248Q and T250Q), phenylalanine (T248F and T250F), leucine (T248L and T250L), Isoleucine (T248I and T250I), methionine (T248M and T250M), glycine (T248G and T248G), phenylalanine (T248F and T250F), tryphophan (T248W and T250W), proline (T248P and T250P), or valine (T248V and T250V), preferably an alanine (T248A and T250A).

The polynucleotide of the medical use of the invention can be comprised in an expression cassette, comprising a promoter, which can be a ubiquitous promoter or a tissue specific promoter and in a particular embodiment of the invention, a neuron-specific promoter, said cassette further comprising a nucleic acid molecule comprising a nucleotide sequence that encodes a mutant form of the human E2F4 transcription factor in which positions 248 and/or 250 are mutated so that they cannot be phosphorylated. Hence, in another particular embodiment of the medical use of the invention, the polynucleotide is comprised in an expression cassette.

The term "expression cassette" as used herein refers to a distinct component of DNA which comprises a gene and a regulatory sequence, such as a promoter, which allows the expression cassette to produce RNA and protein when present in the desired host cell. The term "promoter" as used herein refers to a region of DNA upstream of a gene where relevant proteins (such as RNA polymerase and transcription factors) bind to initiate transcription of that gene.

As used herein, the term "ubiquitous promoter" means a promoter which induces the expression of a downstream located gene in any cell type comprising it, i.e. in every cell comprising it regardless of cell type, of tissue or organ location and of function of said cell. The term "tissue-specific promoter", as used herein, refers to a promoter, which controls and directs expression of a gene in a tissue-specific manner, e.g., in brain tissue, in muscle tissue, in liver tissue, in kidney tissue, etc. A brain-specific promoter is a promoter which controls ad directs expression of a gene in brain tissue.

The term "neuron-specific promoter", as described herein, refers to a promoter that may be suitably used in increasing the expression of a foreign nucleic acid and/or a gene, such as the polynucleotide comprised in the expression cassette, in neuron cells, such as brain cells.

Preferably, said neuron-specific promoter is selected from the group consisting of Synapsin, Neuron-Specific Enolase (NSE), human synapsin 1, CaMKII kinase, tubulin alpha (Tα1), and platelet-derived growth factor-beta chain (PDGF). In a particular embodiment of the medical use of the invention, the promoter is selected from the group consisting of the human Synapsin 1 promoter, the CaMKIIα promoter, the NSE (Neuron-Specific Enolase) promoter and the Tα1 promoter. In a more particular embodiment of the medical use of the invention, the promoter is human Synapsin 1 promoter.

The term "human Synapsin 1 promoter" includes any promoter comprising a functional portion of the human synapsin 1 gene. For example, the promoter may be a human synapsin 1 (hSYNI) promoter, a human synapsin 1 with 5' extension promoter (e.g. a nucleotide sequence according to SEQ ID NO: 3), a human synapsin 1 with 3' extension promoter (e.g. a nucleotide sequence according to SEQ ID NO: 4) an eSYN promoter (e.g. a nucleotide sequence according to SEQ ID NO: 5) or a truncated human synapsin 1 promoter (e.g. a nucleotide sequence according to SEQ ID NO: 6). The hSYN 1 promoter is well known to those skilled in the art. The promoter comprising a functional portion of the human synapsin 1 gene may additionally comprise an enhancer element, which may be any enhancer element. For example, the eSYN promoter is a hybrid promoter containing the human synapsin 1 promoter and a CMV enhancer. In a particular embodiment of the medical use of the invention, the promoter is the Synapsin 1 promoter and comprises a nucleotide sequence selected from SEQ ID NO: 2 - 6.

In another particular embodiment of the medical use of the invention, the promoter is the CaMKIIa promoter according to SEQ ID NO: 7. In another particular embodiment of the medical use of the invention, the promoter is the NSE (Neuron-Specific Enolase) promoter according to SEQ ID NO: 8. In another particular embodiment of the medical use of the invention, the promoter is the Tα1 promoter according to SEQ ID NO: 9.

The polynucleotide of the medical use of the invention can be comprised in a vector. Hence, in another particular embodiment of the medical use of the invention, the polynucleotide is comprised in an expression vector.

The term "vector", as used herein, refers to a vehicle through which a polynucleotide or a DNA molecule can be manipulated or introduced into a cell. The vector can be a linear or circular polynucleotide, or it can be a larger polynucleotide or any other type of construction such as the DNA or RNA of a viral genome, a virion or any other biological construct that allows the manipulation of DNA or its introduction in a cell. It is understood that the terms "recombinant vector", "recombinant system" can be used interchangeably with the term vector. A person skilled in the art will understand that there is no limitation as regards the type of vector, which can be used because said vector can be a cloning vector suitable for propagation and for obtaining the polynucleotides or suitable gene constructs or expression vectors in different heterologous organisms suitable for purifying the polynucleotides of the invention. Thus, suitable vectors according to the present invention include expression vectors in prokaryotes such as pET (such as pET14b), pUC18, pUC19, Bluescript and their derivatives, mp18, mp19, pBR322, pMB9, ColEl, pCRI, RP4, phages and shuttle vectors such as pSA3 and pAT28, expression vectors in yeasts such as vectors of the type of 2 micron plasmids, integration plasmids, YEP vectors, centromeric plasmids and the like, expression vectors in insect cells such as the pAC series and pVL series vectors, expression vectors in plants such as vectors of expression in plants such as plBl, pEarleyGate, pAVA, pCAMBIA, pGSA, pGWB, pMDC, pMY, pORE series vectors and the like and expression vectors in superior eukaryotic cells based on viral vectors (adenoviruses, viruses associated to adenoviruses as well as retroviruses and lentiviruses) as well as non-viral vectors such as pSilencer 4.1-CMV (Ambion), pcDNA3, pcDNA3.1/hyg pHCMV/Zeo, pCR3.1, pEFI/His, pIND/GS, pRc/HCMV2, pSV40/Zeo2, pTRACER-HCMV, pUB6/V5-His, pVAXI, pZeoSV2, pCI, pSVL and pKSV-10, pBPV-1, pML2d and pTDTI.

The term "expression vector" as used herein is a plasmid or virus designed for gene expression in cells. The expression vector is used to introduce a specific gene of interest into a target cell and can commandeer the cell's mechanism to produce the protein encoded by the gene. The vector of the invention can be used to transform, transfect, or infect cells that can be transformed, transfected or infected by said vector. Said cells can be prokaryotic or eukaryotic. Said vector can be obtained by conventional methods known by the persons skilled in the art (Sambrook et al., 2001, "Molecular cloning, to Laboratory Manual", 2nd ed., Cold Spring Harbor Laboratory Press, N.Y. Vol 1-3 a).

In a particular embodiment of the medical use of the present invention, the plasmid vector is a mammalian expression vector. In a more particular embodiment of the medical use of the invention, the expression vector is a viral vector.

The term "viral vector" as used herein refers to a virus particle that has been modified based on a natural virus or the like to remove pathogenicity and to include a region for introducing a foreign gene. Furthermore, in the present invention, the term "viral vector" refers not only to a virus genome (nucleic acid form)-containing particle, but also to a virus-like particle that does not contain a viral genome, i.e., a hollow particle. It is preferable to obtain a "viral vector" that contains a viral genome (nucleic acid form) inside the particle. Viral vectors can be used for genetic manipulation (i.e., "cloning vectors") for the introduction/transfer of nucleic acid into cells and for transcription or translation of the inserted nucleic acid in the cell, working as "expression vectors". Examples of viral expression vectors are adenovirus associated virus (AAV), adenoviruses (Ads) and retroviruses (including lentiviruses).

In a particular embodiment of the medical use of the invention, the viral vector is an adeno-associated virus vector.

The term "adeno-associated virus", or its acronym "AAV" refers to members of the class of viruses belonging to the genus *Dependoviridae* of the family *Parvoviridae.* Adeno-associated virus is a single-stranded DNA virus that grows exclusively in cells, where certain functions are provided by a co-infecting helper virus. All AAV serotypes exhibit very similar replication characteristics mediated by homologous rep genes; and all carry three related capsid proteins. At least 13 naturally occurring AAV serotypes, numbered sequentially, are known in the art. In a particular embodiment of the medical use of the invention, the AAV vector is selected from the serotype AAV1, AAV2, AAV3, AAV4, AAV5, AAV6, AAV7, AAV8, AAV9, AAV10, AAV11, AAV12, or AAV13. In a more particular embodiment of the medical use of the invention, the AAV vector is selected from the serotype AAV2, AAV8, or AAV9, preferably AAV9. According to some embodiments, the AAV vector is an AAV9.

In a particular embodiment of the medical use of the invention, the adeno-associated virus vector is a self-complementary AAV.

The expression "self-complementary adeno-associated virus" or its acronym "scAAV", as used herein refers to a viral vector engineered from the naturally occurring adeno-associated virus (AAV) to be used as a tool for gene therapy. This lab-made progeny of recombinant AAV (rAAV) is termed "self-complementary" because the coding region has been designed to form an intra-molecular double-stranded DNA template. A rate-limiting step for the standard AAV genome involves the second-strand synthesis since the typical AAV genome is a single-stranded DNA template. However, this is not the case for scAAV genomes. Upon infection, rather than waiting for cell mediated synthesis of the second strand, the two complementary halves of scAAV will associate to form one double stranded DNA (dsDNA) unit that is ready for immediate replication and transcription.

The expression of polynucleotide comprised in the viral vector is normally controlled by a promoter, which can be a ubiquitous promoter or a tissue specific promoter. In a particular embodiment of the medical use of the invention, the polynucleotide within the vector is operably controlled by a neuron-specific promoter.

Preferably, said neuron-specific promoter is selected from the group consisting of Synapsin, Neuron-Specific Enolase (NSE), human synapsin 1, CaMKII kinase, tubulin alpha (Tα1), and platelet-derived growth factor-beta chain (PDGF). In a particular embodiment of the medical use of the invention, the promoter is selected from the group consisting of the human Synapsin 1 promoter, the CaMKIIα promoter, the NSE (Neuron-Specific Enolase) promoter and the Tα1 promoter. In a more particular embodiment of the medical use of the invention, the promoter is human Synapsin 1 promoter.

In a particular embodiment of the medical use of the invention, the promoter is the Synapsin 1 promoter and comprises a nucleotide sequence selected from SEQ ID NO: 2 - 6. In another particular embodiment of the medical use of the invention, the promoter is the CaMKIIa promoter according to SEQ ID NO: 7. In another particular embodiment of the medical use of the invention, the promoter is the NSE (Neuron-Specific Enolase) promoter according to SEQ ID NO: 8. In another particular embodiment of the medical use of the invention, the promoter is the Tα1 promoter according to SEQ ID NO: 9.

The polynucleotide of the medical use of the invention finds use in the treatment of AD. As used herein, the terms "to treat", "treating" or "treatment" refer, and include without limitation, therapeutic treatment, the purpose of which is to reverse, restraining, slowing, reduce, suppress, delay or stop the progression or severity of an existing symptom, clinical sign, disorder, or condition associated with the disease or disorder. The term "treatment" includes reducing or alleviating at least one adverse effect or condition of an ailment, a disease or disorder, such as an AD and the severity of said condition associated to the disease or disorder. Treatment is usually "effective" when at least one or more symptoms or clinical signs are reduced. Alternatively, treatment is "effective" if disease progression is delayed or halted. That is, "treatment" includes not only the improvement of symptoms or clinical signs associated to the disease, but also the interruption of at least a condition that indicates the progression or worsening of symptoms or clinical signs that would be expected in the absence of treatment. The beneficial or desirable clinical outcome, whether detectable or not, is a reduction in at least one or more symptoms or clinical signs, a reduction in the extent of the disease, a reduction in the duration or frequency of the condition associated to the disease, a stabilization (not aggravated) condition of the disease. These include, but are not limited to, delayed or slowed progression, amelioration or alleviation of the disease state, and remission (partial or total), including protection from the disease. The term "treatment" of a disease also includes providing relief from symptoms, clinical signs or side effects of the disease (including symptomatic treatment). In the present context, the polynucleotide of the invention is to be used in the treatment of Alzheimer's disease (AD) in a patient diagnose as having AD.

The term "Alzheimer's disease" or its acronym "AD" as used herein refers to a mental impairment associated with a specific degenerative brain disease which is characterized by the appearance of senile plaques, neurofibrillary tangles and progressive neuronal loss clinically manifested as progressive memory deficiencies, confusion, behavioral problems, inability to take care of oneself, gradual physical deterioration, and ultimately death. In a particular embodiment of the medical use of the invention, Alzheimer's disease is a disease in any of the stages according to the Braak scale:
- Stages I-II: the brain area affected by the presence of neurofibrillary tangles corresponds to the transentorhinal region of the brain
- Stages III-IV: the affected brain area also extends to areas of the limbic region, such as the hippocampus
- Stages V-VI: the affected brain area also involves the neocortical region.

This classification by neuropathological stages is correlated with the clinical progression of the disease, there being parallelism between memory decline with neurofibrillary changes and the formation of neuritic plaques in the entorhinal cortex and hippocampus (stages I to IV). Likewise, the isocortical presence of these changes (stages V and VI) is correlated with clinically severe alterations. The transentorhinal stage (I-II) corresponds to clinically silent periods of the disease. The limbic stage (III-IV) corresponds to a clinically incipient AD. The neocortical stage corresponds to a fully developed AD.

The expression "diagnosed as having AD" refers to the process of diagnosing a subject as suffering from AD by a physician or a medically qualified person, through the use of any required exams, such as medical history, mental status tests, physical and neurological exams, diagnostic tests and brain imaging.

According to the first aspect of the invention, the patient diagnosed with AD also has mild, moderate or severe dementia .

As used herein the term "dementia" refers to a person's ability to think, learn, remember, use judgement, and make decisions. Signs of dementia include memory loss and trouble concentrating, completing tasks, understanding, remembering, following instructions, and solving problems. Other common signs may include changes in mood or behavior, loss of motivation, and being unaware of surroundings. Dementia may be mild, moderate or severe. There are many causes of dementia, chiefly amongst them AD. The level of dementia can be determined through the clinical dementia rating.

As used herein, the term "clinical dementia rating" or its acronym "CDR" is used to determine the "dementia" of a subject and possibly determine its treatment. CDR is a global assessment instrument used in clinical trials and longitudinal research projects to rate the presence and severity of cognitive problems in Alzheimer disease and related disorders. The CDR rates the severity of Alzheimer disease using a 5-point scale that rates the severity of signs and symptoms as they affect the patient's ability to function in the 6 cognitive categories of memory, orientation, judgment and problem solving, community affairs/involvement, home life and hobbies, and personal care. Although designed for use with patients with a probable diagnosis of AD, the CDR is appropriate for use in the assessment of dementia that is associated with other medical disorders (e.g., Lewy body disease). The CDR rating varies as follows:
- 0=Normal (CDR0)
- 0.5=Very Mild Dementia (CDR0.5)
- 1=Mild Dementia (CDR1)
- 2=Moderate Dementia (CDR2)
- 3=Severe Dementia (CDR3)

The CDR is completed by the researcher or a clinician after performing a face-to-face, semi-structured interview with the patient and a reliable informant such as a spouse or other family member/caregiver. The informant is interviewed first, and the results of that interview are used to assess patient recall of events and to confirm the accuracy of patient responses. In each cognitive category the patient receives a score of 0 (no dementia) to 3 (severe dementia). The CDR can be scored to obtain a global score by using an algorithm that weights memory more heavily than the other categories, or it can be scored using the sum of boxes (SOB) method in which all categories are weighted equally; in general, the higher the score, the greater the severity of dementia.

In a particular embodiment of the medical use of the invention, the patient is a patient diagnosed as having Alzheimer's Disease and having mild to moderate dementia and/or a CDR of 1-2. In a particular embodiment of the medical use of the invention, the patient is a patient diagnosed as having Alzheimer's Disease and having moderate to severe dementia n/and/or a CDR of 2-3. In a particular embodiment of the medical use of the invention, the patient is a patient diagnosed as having Alzheimer's Disease and having mild dementia and/or a CDR of 1. In a particular embodiment of the medical use of the invention, the patient is a patient diagnosed as having Alzheimer's Disease and having moderate dementia and/or a CDR of 2. In a particular embodiment of the medical use of the invention, the patient is a patient diagnosed as having Alzheimer's Disease and having severe dementia and/or a CDR of 3.

The CDR can be performed or administered and interpreted in any setting in which a patient requires evaluation or treatment for AD or other dementia, including inpatient and outpatient healthcare settings and community settings (e.g., during provision of in-home care by a home health nurse; as part of a public health screening program).

The polynucleotide according to the medical use of the invention can be administrated by several means which are known to the expert in the field, such as parenterally (e.g. intramuscular, intraperitoneal, intravenous, or subcutaneous injection), by intrathecal intracerebroventricular injection or by injection into the cisterna magna. In a particular embodiment of the medical use of the invention, the polynucleotide of the invention is administered into the cisterna magna or by intravenous route.

The term "administered into the cisterna magna" as used herein refers to the administration of the polynucleotide into the cisterna magna (posterior cerebellomedullary cistern, or cerebellomedullary cistern) which is the largest of the subarachnoid cisterns. The cisterna magna occupies the space created by the angle between the caudal/inferior surface of the cerebellum, and the dorsal/posterior surface of the medulla oblongata (it is created by the arachnoidea that bridges this angle). The fourth ventricle communicates with the cistern via the unpaired midline median aperture. It is continuous inferiorly with the subarachnoid space of the spinal canal.

In a particular embodiment of the medical use of the invention, the patient is a mammal, preferably a human of any race and gender.

### Pharmaceutical composition and administration dosages

The polynucleotide of the medical use of the invention can be administrated to a subject in the form of a pharmaceutical composition.

In one aspect, the present invention provides a pharmaceutical composition for use in the treatment of Alzheimer's Disease in a patient diagnosed as having Alzheimer's Disease and exhibiting mild to severe dementia. Said pharmaceutical composition comprises an effective amount of a polynucleotide encoding a mutant form of the human E2F4 transcription factor, wherein one or more amino acid residues at positions corresponding to 248 and/or 250 of the wild-type human E2F4 transcription factor are mutated such that said positions cannot be phosphorylated.

The polynucleotide as referred to herein may, in some embodiments, be an isolated nucleic acid molecule that itself forms or is comprised within an expression cassette, for example, a transcription cassette. Such an expression cassette is designed for expression of the mutant form of the human E2F4 transcription factor and typically comprises regulatory elements, including for instance a promoter adapted for expression in a mammalian neuron (such as a neuron-specific promoter), operably linked to the nucleotide sequence encoding said mutant form of the human E2F4 transcription factor.

The pharmaceutical composition of the invention is suitable for delivering said polynucleotide (which may be, for example, in the form of an expression cassette as described above) to target cells, such as neurons within the patient's brain. To achieve this delivery, the pharmaceutical composition may employ various delivery strategies.

In some embodiments, the polynucleotide or expression cassette is comprised within a viral vector. Examples of suitable viral vectors include, but are not limited to, an adeno-associated virus (AAV) vector, a lentiviral vector, or other viral vectors known in the art to be suitable for gene delivery, particularly to neuronal cells.

In other embodiments, the pharmaceutical composition utilizes non-viral nucleic acid delivery methods. Such non-viral methods may involve formulating the polynucleotide or expression cassette with one or more non-viral delivery agents. Examples of such agents and formulations include, but are not limited to, lipid nanoparticles (LNPs), liposomes, polymeric nanoparticles, peptide-based delivery systems, or dendrimers. Non-viral delivery may also encompass methods such as electroporation, sonoporation, or other physical methods for facilitating nucleic acid entry into target cells, when the polynucleotide or expression cassette is appropriately formulated for such procedures.

The pharmaceutical composition, whether employing viral or non-viral delivery methods, or delivering an expression cassette or other form of the polynucleotide, may further comprise one or more pharmaceutically acceptable carriers, diluents, buffers, stabilizers, or excipients appropriate for the specific formulation, the chosen delivery technology, and the intended mode of administration

Another aspect of the present invention relates to a pharmaceutical composition comprising an adeno-associated virus serotype 9 (AAV9) vector which comprises a polynucleotide that encodes a mutant form of the human E2F4 transcription factor in which positions 248 and/or 250 are mutated so that they cannot be phosphorylated for use in the prevention and/or treatment of Alzheimer's disease wherein the pharmaceutical composition is administered to achieve between 0.5 × 10¹⁴ and 6 × 10¹⁴ viral genomes (vg) of the adeno-associated virus serotype 9 (AAV9) vector per kg of brain, form here onwards the pharmaceutical composition use of the invention.

All definitions and particular embodiments disclosed previously in relation to the medical use of the invention are equally valid for the pharmaceutical compositions of the invention.

The pharmaceutical composition use of the invention may further comprise an excipient and/or an acceptable adjuvant.

The term "excipient" refers to a vehicle, or diluent that is administered with the active ingredient and includes solvents, dispersion media, coatings, stabilizing agents, diluents, preservatives, antibacterial and antifungal agents, isotonic agents, adsorption-delaying agents, and the like. Such pharmaceutical excipients can be sterile liquids, such as water and oils, including those of petroleum, animal, vegetable or synthetic origin, such as peanut oil, soybean oil, mineral oil, sesame oil and similars. Water or saline aqueous solutions and aqueous dextrose and glycerol solutions, particularly for injectable solutions, are preferably used as vehicles. Suitable pharmaceutical vehicles are described in Remington: The Science and Practice of Pharmacy, 21st Ed. Philadelphia, PA. Lippincott Williams & Wilkins (2005); or by Rowe et al., Handbook of Pharmaceutical Excipients, Pharmaceutical Press, 6th Ed. (2009).

In a particular embodiment of the pharmaceutical composition use of the invention, the pharmaceutical composition is formulated using dextrose solution in phosphate-buffered saline (PBS) and wherein the dextrose is present at a concentration that provides an osmolarity to the pharmaceutical composition similar to that of the human Cerebrospinal Fluid (CSF).

The term "phosphate-buffered saline" or "PBS" as used herein refers to a water-based salt solution containing disodium hydrogen phosphate, sodium chloride and, in some formulations, potassium chloride and potassium dihydrogen phosphate. The buffer helps to maintain a constant pH, and the osmolarity and ion concentrations of the solutions are isotonic, meaning they match those of the human body.

The term "osmolarity" or "osmotic concentration" as used herein refers to the measure of solute concentration, defined as the number of osmoles (Osm) of solute per litre (L) of solution (osmol/L or Osm/L). The osmolarity of a solution is usually expressed as Osm/L (pronounced "osmolar"), in the same way that the molarity of a solution is expressed as "M" (pronounced "molar"). In the present invention the osmolarity refers to the osmolarity of the CSF. The term "Cerebrospinal Fluid" or its acronym "CSF" as used herein refers to a clear, colorless transcellular body fluid found within the meningeal tissue that surrounds the vertebrate brain and spinal cord, and in the ventricles of the brain. CSF has an average CSF osmolarity of 280 to 295 mOsm/L.

In a particular embodiment of the pharmaceutical composition use of the invention, the dextrose is present at a concentration that provides an osmolarity of between 280 to 295 mOsm/L, preferably of about 280 mOsm/L, 281 mOsm/L, 282 mOsm/L, 283 mOsm/L, 284 mOsm/L, 285 mOsm/L, 286 mOsm/L, 287 mOsm/L, 288 mOsm/L, 289 mOsm/L, 290 mOsm/L, 291 mOsm/L, 292 mOsm/L, 293 mOsm/L, 294 mOsm/L, 295 mOsm/L.

In a particular embodiment of the pharmaceutical composition use of the invention, the dextrose is present at a concentration of between 4.0 mM to about 5.0 mM. In another particular embodiment of the pharmaceutical composition use of the invention, the dextrose is present at a concentration of about 4 mM, 4.1 mM, 4.2 mM, 4.3 mM, 4.4 mM, 4.5 mM, 4.6 mM, 4.7 mM, 4.8 mM, 4.9 mM, or 5 mM. In yet another particular embodiment of the pharmaceutical composition use of the invention, the dextrose is present at a concentration of 4.4 mM.

In a particular embodiment, the pharmaceutical composition use of the invention further comprises a surfactant. The term "surfactant" as used herein refers to any substance that lowers the surface tension of the pharmaceutical composition of the invention. In a particular embodiment, the pharmaceutical composition use of the invention further comprises a surfactant selected from a group consisting of: sodium lauryl sulfate, polysorbate 80, poloxamer, lauryl macrogolglycerides, polyoxyethylene alkyl ethers, and any combination thereof. In a more particular embodiment, the surfactant is poloxamer, preferably poloxamer 188.

The term "poloxamer" as used herein refers to nonionic triblock copolymers composed of a central hydrophobic chain of polyoxypropylene (poly(propylene oxide)) flanked by two hydrophilic chains of polyoxyethylene (poly(ethylene oxide)). Because the lengths of the polymer blocks can be customized, many different poloxamers exist that have slightly different properties. For the generic term poloxamer, these copolymers are commonly named with the letter P (for poloxamer) followed by three digits: the first two digits multiplied by 100 give the approximate molecular mass of the polyoxypropylene core, and the last digit multiplied by 10 gives the percentage polyoxyethylene content (e.g. P407 = poloxamer with a polyoxypropylene molecular mass of 4000 g/mol and a 70% polyoxyethylene content). For the Pluronic and Synperonic tradenames, coding of these copolymers starts with a letter to define its physical form at room temperature (RT) (L = liquid, P = paste, F = flake (solid)) followed by two or three digits, The first digit (two digits in a three-digit number) in the numerical designation, multiplied by 300, indicates the approximate molecular weight of the hydrophobe; and the last digit x 10 gives the percentage polyoxyethylene content (e.g., L61 indicates a polyoxypropylene molecular mass of 1800 g/mol and a 10% polyoxyethylene content). In the example given, poloxamer 181 (P181) = Pluronic L61 and Synperonic PE/L 61.

In another particular embodiment of the pharmaceutical composition use of the invention, the dextrose solution in PBS comprises 8.1 mM Na₂HPO₄·7H₂O, 1.5 mM K₂HPO₄, 2.7 mM KCI, 137 mM NaCl, 0.5 mM MgCl₂, 0.9 mM CaCl₂, 4.4 mM Dextrose, wherein the composition further comprises a poloxamer 188 as a surfactant at a concentration of 0.001%.

In a particular embodiment of the pharmaceutical composition use of the invention, the pharmaceutical composition is administrated to achieve about 0.5 × 10¹⁴ vg/kg, 1 × 10¹⁴ vg/kg, 1.5 × 10¹⁴ vg/kg, 2 × 10¹⁴ vg/kg, 2.5 × 10¹⁴ vg/kg, 3 × 10¹⁴ vg/kg, 3.5 × 10¹⁴ vg/kg, 4 × 10¹⁴ vg/kg, 4.5 × 10¹⁴ vg/kg, 5 × 10¹⁴ vg/kg, 5.5 × 10¹⁴ vg/kg, 6 × 10¹⁴ vg/kg of brain.

In another particular embodiment of the pharmaceutical composition use of the invention, the pharmaceutical composition is administered to achieve between 1.21 × 10¹⁴ and 4.86 × 10¹⁴ vg/kg of brain.

The term "viral genomes" as used herein refers to the number of viral particles containing the viral genome which carries the expected polynucleotide. In the present context, the expected polynucleotide corresponds to the polynucleotide of the medical use of the invention. Methods to determine the number of viral genomes are well known in the art, examples such as, without limitation, RT-PCR, qPCR, etc.. The term "viral genomes" as used herein, only includes viral particles which contain the expect polynucleotide, but not viral particles which contain only partial polynucleotides or which are empty, nor does it include viral particles which contain wild type viral genomes or viruses.

In a particular embodiment of the pharmaceutical composition of the invention the content of viable viral genomes within the pharmaceutical composition is of 35-50% with respect to the total number of viral particles. In another particular embodiment of the pharmaceutical composition of the invention the content of viable viral genomes within the pharmaceutical composition is of about 35%, 37.5%, 40%, 42.5%, 45%, 47.5%, 50% with respect to the total number of viral particles. In yet another particular embodiment of the pharmaceutical composition of the invention the content of viable viral genomes within the pharmaceutical composition is of about 40% with respect to the total number of viral particles.

The pharmaceutical composition use of the invention refers to the medical use of the composition for preventing and/or treatment of AD, wherein the term "treatment" has been previously defined.

The terms "prevention", "to prevent", "preventing" or "prophylaxis", as used herein, include, without limitation, the capacity to prevent, decrease, reduce, minimize, or delay the onset or development of a disease or condition before its onset. It also refers ameliorating the risk of a symptom, clinical sign, disorder, condition, or disease. The term prevention also includes protecting the subject from a symptom, disorder, condition, or disease. The immunogenic and vaccine compositions of the medical use of the invention are for use in a method of prevention and may be applied or administered prophylactically.

A pharmaceutical composition can be administered through several routes such as parenterally (e.g. intramuscular, intraperitoneal, intravenous, or subcutaneous injection), by intrathecal intracerebroventricular injection or by injection into the cisterna magna, as previously described. In a particular embodiment of the pharmaceutical composition use of the invention, the composition is administered intracisternally. The term "instracisternally" as used herein refers to the administration into the cisterna magna (see above). The expert in the field will be aware of the techniques and rightful way of performing such administration in a safely fashion.

In a particular embodiment of the pharmaceutical composition use of the invention, the composition is a single dose composition. The term "single dose" as used herein refers to a pharmaceutical composition which is formulated intended for a single use, being that the single dose when administered to the subject is effective to achieve a therapeutic and/or prophylactic effect against AD as previously defined. This means that a single dose is effective for treating, preventing, reducing, controlling and/or ameliorating clinical signs associated to AD.

The single dose as previously defined, can be administered to the patient when necessary, but is preferably administered only once in the patient's lifetime. In a particular embodiment of the pharmaceutical composition use of the invention, the composition is administered only once within the lifetime of the patient.

The present invention is further defined in the following aspects:
1. A polynucleotide encoding a mutant form of the human E2F4 transcription factor in which positions 248 and/or 250 are mutated so that they cannot be phosphorylated for use in the treatment of Alzheimer's Disease in a patient diagnosed as having Alzheimer's Disease and having mild, moderate or severe dementia.
2. The polynucleotide for use according to aspect 1 wherein the positions 248 and/or 250 contain alanine residues.
3. The polynucleotide for use according to any one of aspects 1 to 2 wherein the polynucleotide is comprised in an expression vector.
4. The polynucleotide for use according to aspect 3, wherein the expression vector is a viral vector.
5. The polynucleotide for use according to aspect 4 wherein the viral vector is an adeno-associated virus vector.
6. The polynucleotide for use according to aspect 5, wherein the adeno-associated virus vector is AAV9.
7. The polynucleotide for use according to aspects 5 or 6, wherein the adeno-associated virus vector is a self-complementary AAV.
8. The polynucleotide for use according to any one of aspects 3 to 7 wherein the polynucleotide within the vector is operably controlled by a neuron-specific promoter.
9. The polynucleotide for use according to aspect 8, wherein the promoter is selected from the group consisting of the human Synapsin 1 promoter, the CaMKIIa promoter, the NSE (Neuron-Specific Enolase) promoter and the Tα1 promoter.
10. The polynucleotide for use according to aspect 9, wherein the promoter is human Synapsin 1 promoter.
11. The polynucleotide for use according to any one of aspects 1 to 9, wherein the polynucleotide is administered into the cisterna magna or by intravenous route.
12. A pharmaceutical composition comprising an adeno-associated virus serotype 9 (AAV9) vector which comprises a polynucleotide that encodes a mutant form of the human E2F4 transcription factor in which positions 248 and/or 250 are mutated so that they cannot be phosphorylated for use in the prevention and/or treatment of Alzheimer's disease wherein the pharmaceutical composition is administered to achieve between 0.5 × 10¹⁴ and 6 × 10¹⁴ viral genomes (vg) of the adeno-associated virus serotype 9 (AAV9) vector per kg of brain.
13. The pharmaceutical composition for use according to aspect 13 wherein pharmaceutical composition is administered to achieve between 1.21 × 10¹⁴ and 4.86 × 10¹⁴ vg/kg of brain.
14. The pharmaceutical composition for use according to any one of aspects 12 or 13, wherein the content of viable viral genomes within the pharmaceutical composition is of 35-50% with respect to the total number of viral genomes.
15. The pharmaceutical composition for use according to aspect 14, wherein the content of viable viral genomes within the pharmaceutical composition is of about 40 % of the total number of viral genomes.
16. The pharmaceutical composition for use according to any one of aspects 12 to 15, wherein the positions 248 and/or 250 within the mutant form of the E2F4 transcription factor contain alanine residues.
17. The pharmaceutical composition for use according to any one of aspects 12 16 wherein the polynucleotide that encodes the mutant form of the E2F4 transcription factor is operably controlled by a neuron-specific promoter.
18. The pharmaceutical composition for use according to aspect 17 wherein the neuron-specific promoter is selected from the group consisting of the human Synapsin 1 promoter, the CaMKIIα promoter, the NSE (Neuron-Specific Enolase) promoter and the Tα1 promoter.
19. The pharmaceutical composition for use according to aspect 18, wherein the promoter is the human Synapsin 1 promoter.
20. The pharmaceutical composition for use according to any of aspects 12 to 19 wherein the pharmaceutical composition is formulated using dextrose solution in PBS and wherein the dextrose is present at a concentration that provides an osmolarity to the pharmaceutical composition similar to that of the human CSF.
21. The pharmaceutical composition for use according to any of aspects 12 to 20 wherein the composition is administered intracisternally.
22. The pharmaceutical composition for use according to any of aspects 12 to 21 wherein the composition is a single dose composition
23. The pharmaceutical composition for use according to any of aspects 12 to 22 wherein the composition is administered only once within the lifetime of the patient

The invention is described below by means of the following examples which are to be construed as merely illustrative and not limitative of the scope of the invention.

### EXAMPLES

### Example 1

### Methods

### Mice

Male and female mice aged 1.5-7 months old were used in this study. Animals were housed under standard conditions of temperature (22 ± 1 °C) and humidity (40-60%) and on a 12 h light/dark cycle. All biosafety procedures and animal care protocols were approved by the animal bioethics committee from the Consejo Superior de Investigaciones Cientificas (CSIC) and the Autonomous Government of Madrid, in compliance with the Spanish and European Union guidelines. Control C57BL/6J mice were purchased from ENVIGO. Double transgenic mice in C57BL/6J genetic background expressing under the control of the Thy1 promoter both mutant human Aβ precursor protein 695 (APP695) with the Swedish (K670N, M671L), Florida (I716V), and London (V717I) FAD mutations and human presenilin 1 (PS1) harboring the M146L and L286V FAD mutations (Tg6799 or 5xFAD mice) (Oakley et al,. J Neurosci. 2006;26(40):10129-40) were purchased from The Jackson Laboratory (strain #008730). These mice were PCR genotyped as indicated by The Jackson Laboratory. h5xFAD mice were obtained after repeated inbreeding of originally hemizygous mice. h5xFAD mice always yielded genetically homogeneous litters when crossed with wild-type (WT) mice (n=20 litters). Homozygosity was confirmed by quantitative PCR (qPCR) genotyping using specific primers of the human APP transgene [upstream primer: 5'-AGGATGGTGATGAGGTAGAG-3' (SEQ ID NO: 1) (from NM_201414 sequence) and downstream primer complementary to: 5' CTGCTGTTGTAGGAACTCGA-3' (SEQ ID NO: 2) (896-915 bp and 1011-1030 bp from the NM_201414 sequence, respectively)], with the Kcn3a genomic sequence as a reference (López-Sánchez et al., Mol Neurobiol. 2022;59(5):3016-3039). h5xFAD mice were used for behavioral, electrophysiological, stereological and biochemical experiments.

### Viral vectors

Neuronal expression of hE2F4DN was achieved using viral vectors with neural cell tropism and containing the neuron-specific, human *Synapsin 1* (*hSyn1*) promoter (Kügler et al., Mol Cell Neurosci. 2001, 17(1):78-96). The *in vivo* studies were carried out using the adeno-associated vector (AAV) AAV-AS, which derives from AAV serotype 9 (AAV9) (Choudhury et al., Mol Ther. 2016, 24(4):726-35), hereafter referred to as dsAAV.hSyn1.hE2F4DNmyc.WPRE3SL (AAV.hE2F4DNmyc) (Fig. 8a) and dsAAV.hSyn1.hE2F4DN.WPRE3SL (AAV.hE2F4DN) (Fig. 3a). The AAV-AS vector has been shown to efficiently cross the blood-brain barrier of mice and of other non-murine mammals such as felines (Choudhury et al., Mol Ther. 2016, 24(4):726-35). To generate this latter vector, the human E2F4 coding sequence with Thr248Ala/Thr250Ala mutations (hE2F4DN) and containing the Kozak sequence (ACCATGG) and a final stop codon was synthesized (GeneScript) and cloned into the pcDNA 3.1(+) vector (Invitrogen). After confirming its sequence, hE2F4DN was PCR amplified and seamless cloned (Gibson et al., Nat Methods. 2009; 6(5):343-5) into the KD10 plasmid (Packgene), flanked by the *hSyn1* promoter and the mutant woodchuck hepatitis virus post-transcriptional regulatory element (WPRE) sequence (WPRE3SL), which lacks tumorigenic capacity (Schambach et al., Gene Ther. 2006;13(7):641-5). The expected sequence was confirmed by Sanger sequencing. The vector was then manufactured as described by Choudhury et al. (Mol Ther. 2016, 24(4):726-35) by co-transfecting the KD10 transgene plasmid together with the adenoviral helper plasmid pAdDeltaF6 (Plasmid #112867, Addgene), and a 5:1 ratio of the pGG-9.47_VP1,3 #2515 (Plasmid #74237; Addgene) and the pGG-9.47_VP2_AS #2562 (Plasmid #74238; Addgene) packaging plasmids. The final purified product was dialyzed in phosphate-buffered saline (PBS) with additional 0.001% Pluronic F-68. Viral titer was determined by qPCR and confirmed by silver stain. The AAV-AS was administered intravenously (i.v.) through the tail vein to 6-week-old or 13-week-old mice at 3.125×10¹³ vg/kg and their expression was allowed until 6-7 months of age, when they were sacrificed (Fig. 3 b). Levels of protein expression were determined by western blot (WB) (Fig. 6 a, b). For *in vitro* experiments, the serotype 5 adenoviral vector (Ad5) (Davidson et al., Nat Genet. 1993;3(3):219-223) was used to generate Ad5-hSyn1-hE2F4DNmyc-WPRE3SL (hE2F4DNmyc) (Fig. 1a), Ad5-hSyn1-hE2F4DNmyc-IRES-EGFP-WPRE3SL (hE2F4DN), and Ad5-hSyn1-EGFP-WPRE3SL (EGFP) vectors (Viral Vector Production Unit, Autonomous University of Barcelona) (Fig. 2a). These vectors contain the *hSyn1* promoter followed by the specific transgene and the WPRE3SL post-transcriptional regulatory element described above. Ad5.E2F4DN-IRES-EGFP contains the transgenes encoding for hE2F4DNmyc and EGFP, flanking an internal ribosome entry site (IRES) (Ghattas et al., Mol Cell Biol. 1991;11(12):5848-5859) to allow coexpression of both hE2F4DNmyc and EGFP as separate ("non-fused") proteins. In contrast, Ad5.EGFP only contains the gene encoding for EGFP. Hippocampal primary cultures were treated with these viruses (50 infective units/cell) at 14-17 days *in vitro* (DIV) and used for immunocytochemistry or electrophysiological experiments after 3DIV to allow transgene expression.

### Antibodies

For immunocytochemistry, anti-Myc tag antibody (ab9106) (Abcam) was used at 1:250, anti-Myc tag antibody [9E10] (ab32) (Abcam) was diluted 1:250, anti-NeuN mAb clone A60 (MAB377; Merck Millipore) was used at 1:800 dilution, anti-Synapsin I antibody (ab64581) (Abcam) was employed at 1:100, anti-MAP2 antibody (ab5392) (Abcam) was diluted 1:1500, anti-Ankyrin-G, clone N106/36 (Neuromab) was used at 1:150, anti-GFP (04404-84) (Nacalai Tesque) was diluted 1:500, and anti-E2F4 Antibody, clone LLF4-2 (MABE160) (Merk Millipore) was used at 1:400.

For immunohistochemistry the rabbit anti-Myc tag pAb (ab9106; abcam) was used at at 1:500 and the mouse anti-NeuN mAb clone A60 (MAB377; Merck Millipore) was used at 1:1000 dilution.

For WB, anti-Post Synaptic Density Protein 95 antibody, clone 6G6-1C9 (MAB1596) (Merk) was used at 1:3000, anti-β-Amyloid pAb #2454 (Cell Signaling Technology; Danvers, MA) was diluted 1:1000 and anti-Actin antibody, clone C4 (MAB1501R) (Merk) was diluted 1:20,000. Finally, anti-E2F4 (E3G2G) Rabbit mAb (40291) (Cell Signaling Technology) was diluted 1:6,000 for WB.

The following secondary antibodies were used for immunocytochemistry (1:1,000 dilution): Alexa Fluor 488 Goat anti-Mouse IgG (H+L) Highly Cross-Adsorbed Secondary Antibody (A-11029) (Invitrogen), Alexa Fluor 488 Goat anti-Rat IgG (H+L) Cross-Adsorbed Secondary Antibody (A-11006) (Invitrogen), Alexa Fluor 594 Goat anti-Mouse IgG (H+L) Highly Cross-Adsorbed Secondary Antibody (A-11032) (Invitrogen), and Alexa Fluor 647 Goat anti-Chicken IgY (H+L) Secondary Antibody (A-21449) (Invitrogen). Alexa Fluor 647 Goat anti-Mouse IgG (H+L) Highly Cross-Adsorbed Secondary Antibody (A-21236) (Invitrogen) was used at 1:600 for immunohistochemistry, and Alexa Fluor 488 Donkey anti-Rabbit IgG (H+L) Highly Cross-Adsorbed Secondary Antibody (A-21206) (Invitrogen) was used at 1:1,000 for immunocytochemistry and 1:600 for immunohistochemistry. In addition, IRDye^{®} 680RD Goat anti-Mouse IgG Secondary Antibody (926-68070) (LI-COR) and IRDye^{®} 800CW Goat anti-Rabbit IgG Secondary Antibody (926-32211) (LI-COR) were used at 1/15,000 dilution for WB.

### Genomic DNA extraction

Genomic DNA was extracted after overnight (O/N) incubation of the mouse tail tips with 1 ml lysis buffer (100 mM Tris-HCl pH 8.5, 5 mM EDTA, 0.2% SDS, 200 mM NaCl, 0.1 mg/ml Proteinase K) at 55 °C, followed by addition of 440µl de 5M NaCl and incubation on ice for 10 min. Extracts were then centrifuged at 16,000 xg for 15 min at 4 °C, and then cold ethanol was added to the supernatants to reach final concentration of 65%. DNA was pelleted by centrifugation as above and washed twice with cold 70% ethanol followed by an additional centrifugation step. Pellets were dried for 5-10 min at 55 °C and then resuspended in water. Samples were finally incubated at 55 °C and subjected to several cycles of sonication (10 min each) to eliminate viscosity.

### RNA Extraction and cDNA Synthesis

Total RNA was extracted using QIAzol Reagent (Qiagen) and cleaned with the column based Rneasy mini kit (Qiagen, #74104) following manufacturer's instruction. Synthesis of cDNA was performed using SuperScript IV Reverse Transcriptase (ThermoFisher Scientifc) following the manufacturer's instructions.

### qPCR

qPCR genotyping was used to confirm 5xFAD homozygosity. qPCR was performed with SsoAdvanced Universal SYBR Green Supermix (BioRad, Hercules, California, USA), using a 7500 Real-Time PCR equipment (Applied Biosystems, Waltham, Massachusetts, USA).

Reverse transcriptase (RT)-qPCR (qRT-PCR) was performed with the 7500 real-time PCR equipment (Applied Biosystems), using the SsoAdvanced Universal SYBR Green Supermix and specific primers for the house keeping gene Rps18 (qMmuCED0045430) and the human E2F4 gene (qHsaCED0048496). Standard curve method was used to calculate the quantity of both genes and transgene expression levels were relativized to those of Rps18.

### Unbiased stereology

Mice were anesthetized with 1µl/g Pentobarbital (Vetoquinol) injected intraperitoneally, and they were perfused with PBS and then with 4% paraformaldehyde (PFA) prepared in PBS. The brains were then post-fixed overnight in 4% PFA and cryoprotected in a solution of 3% glycerol (Panreac; ref.141339) and 3% ethylene glycol (Panreac) in Phosphate Buffer (0.04 M pH 7.4). Serial coronal sections (50 µm) obtained with a Leica VT1000 S Vibrating blade microtome (Leica) were Nissl stained with 0.1% cresyl violet (Sigma-Aldrich; ref. C5042) and then mounted on histological gelatinized glass slides (Sigma-Aldrich, ref. G2500). For each brain, CA1 hippocampal neurons present in sections at -1.6 to 3.3-3.8 mm from bregma were quantified stereologically according to the Allen Mouse Brain Atlas (https://mouse.brain-map.org/) as a reference. The Stereo Investigator software (Version 5.65, MBF Bioscience) was used both to drive a motorized stage (mac 6000 system, MBF Bioscience) on a dual optical head microscope (Nikon Eclipse 80i) and to mark and measure CA1 hippocampal region at 2x (Nikon Microscope Objective Plan UW 2x/0.06 7.5mm WD) under brightfield optics. Any incomplete stained or torn section was considered a random event, designated missing, and then accounted for in the stereological estimate using the fractionator principle (Stereo Investigator software v5.65, MBF Biosciences). Thereafter, the software sequentially chose counting frames (100×100 µm) randomly in the xyz axes, moving the motorized stage automatically within the previously delimited zones in the CA1 region at 100x (Nikon Microscope Objective Plan Apo VC 100x/1.4 Oil ∞/0.17 DIC N2). Neuronal nuclei were then randomly sampled from the defined CA1 region using optical dissector probes, and a neuronal population was determined using a 2 µm top and bottom guard zone CA1 (counting frame, 15×15; sampling grid, 100x100; dissector height, 30 µm; interval, 6). The average number of sampling sites per animal was 290.5± 14.65. Cells were marked only if their edges were within the dissector area and they did not intersect forbidden lines. The cells were also marked if they came into focus as the optical plane moved through the height of the dissector. The precision of our stereological counts was determined by the Gunderson coefficient of error equation (Sánchez-Puelles et al, Cereb Cortex. 2020;30(2):505-524).). This method of sampling and the section intervals were tested in a pilot experiment to ensure that the number of cells estimated was representative of their total number.

### Hippocampal cultures

Primary hippocampal neurons derived from CD1 mouse embryos of unknown sex were harvested from embryonic day (E)17, and dissociated hippocampal neurons were then prepared as previously described (Walton et al, Sci Rep. 2019;9(1):4594). Briefly, pups were decapitated and brains were placed in cold Hanks' balanced salt solution (HBSS), without calcium or magnesium (Gibco #14185-052) with Hepes (1:100, Gibco ref. 15630-056). Meninges were then removed, and hippocampi were carefully dissected. Hippocampi were incubated for 15 min at 37 °C in HBSS containing 2.5 mg/ml trypsin (Gibco, ref. 1509-046) in a 15 ml conical centrifuge tube, 1 mg/ml DNAse (Roche, ref. 10104159001) was added for the last 3 minutes. After chemical dissociation the reaction was stopped and hippocampi were washed 3 times with 5 ml of HBSS at 37 °C each time. Then, hippocampi were mechanically dissociated by passing them through the enlarged bore of a pasteur pipette 10-15 times. Non-dissociated tissue was allowed to collect at the bottom by gravity and clean supernatant with dissociated cells transferred to a new 15 ml centrifuge tube. 1 ml of HBSS at 37 °C was added to the remaining tissue and dissociated again 10-15 times with a flame polished Pasteur pipette to reduce the diameter of the tip. Non-dissociated tissue was allowed to collect at the bottom by gravity. Clean supernatant with dissociated cells was transferred to the same centrifuge tube in which cells from the first dissociation were collected. Then, HBSS containing hippocampi were centrifuged (300 xg, 5min) and resuspended in plating medium, DMEM (Gibco, ref. 41966-029) containing 10% inactivated Horse Serum (Gibco, ref. 26050088) and penicillin-streptomycin (25 U/ml) (Gibco, ref. 15140-122). Neurons were plated at a density of 15,000 cells (imaging studies) or 30,000 cells (electrophysiology) on 10-mm diameter coverslips (Menzel Glässer, ref. 631-1340) previously coated with poly-L-lysine 1 mg/ml (Sigma-Aldrich ref. P2636, prepared in 150 mM borate buffer, pH 8.4). Neurons were allowed to attach to coverslips for 1 to 2 h in a humidified atmosphere containing 5 % CO₂ at 37 °C. Once neurons were settled, neuronal plating medium was washed with maintenance medium consistent of Neurobasal without L-Glutamine (Gibco, ref. 21103-049) medium supplemented with B27 (Gibco, ref. 17504-044), penicillin-streptomycin (25 U/ml) (Gibco, ref. 15140-122) and GlutaMAX (Gibco, ref. 35050-061). Half of the culture medium was exchanged by fresh maintenance medium every 3-4 days. Imaging and electrophysiological studies were performed at 15-20 DIV.

### Immunocytochemistry and image acquisition

Primary hippocampal cultures were fixed for 15 min with 4% PFA at room temperature and then incubated for 30 min at room temperature (RT) with 0.1% Triton X-100 phosphate buffered saline (PBS-T) containing 10% fetal bovine serum (FBS) (Gibco, ref. 10500064), to block antibody unspecific binding. Coverslips containing neurons were then incubated for 1 h at RT with the primary antibody diluted to the appropriate concentration in PBS-T containing 1% FBS (PBS-T/1% FBS). After 3 washes in PBS-T/1% FBS, incubation was carried out for 1 h at RT in PBS-T/1% FBS containing 100 ng/ml 4',6-diamidino-2-phenylindole (DAPI) (Sigma-Aldrich, ref. D9542) and secondary antibodies. Finally, 3 washes were performed with PBS-T and one with PBS. Coverslips were mounted in ImmunoSelect^{®} Antifading Mounting Medium (Quimigen; SCR-38587). Confocal images were acquired with a Leica SP5 confocal microscope (Figure 1 b-g). E2F4 expression analysis was performed after obtaining images with a Epiflouresce microscope Nikon Eclipse 80i (Figure 2 b, d). Image analysis was performed using ImageJ (Fiji). EGFP and E2F4 labeling were used as markers to determine that every neuron expressing EGFP co-expresses hE2F4DN and to quantify the level of expression of E2F4. To determine Ad5. vector transduction efficacy, EGFP and NeuN coexpression was analyzed. Fluorescence intensity was measured by manually tracing line plots surrounding the soma in the 488 or 594-channel image, for EGFP or NeuN expression respectively, and using the region of interest (ROI) plugin from the Fiji software to obtain a mask. This mask was superimposed over E2F4 or EGFP images, 594 or 488-channel respectively, to measure the intensity for E2F4 expression levels or EGFP expression, to identify transduced neurons. In addition, three more ROIs were randomly drawn to measure the background fluorescence intensity. Imaging experiments were analyzed blindly.

### Immunohistochemistry and image acquisition.

Mice were transcardially perfused with PBS, and then with 4% PFA, and brains were postfixed O/N at 4 °C with 4% PFA. Vibratome obtained 50-µm sections were permeabilized and blocked for 1 h at RT in Blocking Buffer (BB) containing phosphate buffered saline (PBS)/0.2% Triton X-100 (Sigma-Aldrich, ref. 11332481001) (0.1% PBTx) and 10% FBS, and incubated O/N at 4 °C with the primary antibodies in BB. After washing with BB, the sections were incubated for 1 h at RT in BB containing 100 ng/ml DAPI with the secondary antibodies. The sections were washed in three times with 0.1% Triton X-100 PBS and twice with PBS before mounting with ImmunoSelect^{®} Antifading Mounting Medium (Dianova, ref. SCR-038447). Cryosections (15 µm) were permeabilized and blocked for 1 h at RT in phosphate-buffered saline (PBS)/0.1% Triton X-100 (Sigma-Aldrich) (0.1% PBTx) and 10% fetal calf serum (FCS; Invitrogen) and incubated O/N at 4 °C with the primary antibodies in 0.1% PBTx plus 1% FCS. After washing with 0.1% PBTx, the sections were incubated for 1 h at RT in 0.1% PBTx plus 1% FCS with the secondary antibodies. The sections were washed in 0.1% PBTx, and then, they were incubated with 100 ng/ml 4',6-diamidine-2'-phenylindole dihydrochloride (DAPI; Sigma-Aldrich) in PBS before mounting with PBS/glycerol (1:1). Images were obtained with a Leica Thunder Imager DM i8 or SP5 confocal microscope. Counting of Myc-positive -positive nuclei in brain sections was performed using z-projections as described previously (López-Sánchez et al., Neurotherapeutics. 2021;18(4):2484-2503).

### Electrophysiological recordings

### Whole-cell recordings of hippocampal primary cultures.

Electrophysiological experiments were performed in cultured neurons grown for 15-20 DIV on glass coverslips using patch-clamp whole-cell configuration (Barrio-Alonso et al., Sci Rep. 2018;8(1):14316). Transduced neurons were identified by the expression of the EGFP reporter. Cells were visualized with an Olympus BX50WI microscope coupled with a 40x water immersion objective and infrared-DIC optics. The extracellular medium contained (in mM): 119 NaCl, 2.5 KCI, 1 NaH₂PO₄, 26 NaHCO₃, 2.5 CaCl₂, 1.2 MgCl₂, 11 glucose and 100 µM picrotoxin, and was gassed with 95% O₂/5% CO₂ (pH 7.3). The patch pipette contained (in mM): 130 K-gluconate, 10 NaCl, 1 EGTA, 0.133 CaCl₂, 2 MgCl₂, 10 HEPES, 3.5 MgATP, 1 NaGTP (pH 7.4). Picrotoxin is always present in the ACSF, a noncompetitive antagonist of GABA_{A} receptors. The pipette resistance was in the range of 3-5 MΩ. Experiments were performed at 37 °C. Resting membrane potential was estimated in whole-cell current clamp configuration at I = 0 immediately after membrane rupture. APs were evoked by depolarizing current pulses (40 pA steps, 1 s). Spontaneous synaptic transmission was recorded by clamping cells at -70 mV. Currents were obtained with PC-ONE amplifier (Dagan Corporation). Series and input resistances were monitored throughout the experiment using a -5 mV pulse. Recordings where access resistance changed >20% during the experiment were discarded from the analysis. Signals were fed to a Pentium-based PC through a DigiData 1440 interface board (Molecular Devices). The pCLAMP 10.2 software (Molecular Devices, RRID: SCR_011323) was used for data display, acquisition, and storage.

### Ex-vivo Field Recordings

To prepare acute slices, mice were cervical dislocated and decapitated. The brain was rapidly removed to ice-cold, oxygenated, dissection solution (in mM): 10 D-Glucose, 4 KCl, 26 NaHCO₃, 233.7 sucrose, 5 MgCl₂, 1:1,000 Phenol Red.. Coronal hippocampal slices (300 µm) were made with a vibratome (LeicaVT1200S), and then they were transferred to a recovery chamber containing artificial cerebrospinal fluid (CSF), ACSF, (in mM): 119 NaCl, 2.5 KCI, 1 NaH₂PO₄, 11 glucose, 26 NaHCO₃, 1.2 MgCl₂, 2.5 CaCl₂at 24-26 °C for at least 1.5 h before recording. For field recording, field excitatory postsynaptic potentials (fEPSPs) were recorded from the stratum radiatum of CA1 with glass microelectrodes (700 kΩ-1 MΩ resistance) filled with aCSF, used also as external solution. To assess the function of excitatory synapses, electrophysiological recordings were performed in the presence of picrotoxin (100 µM), to block GABA_{A} receptor responses. fEPSPs were evoked by stimuli (100 µs) delivered through a concentric bipolar electrode (FHC, ref. CBBRE75), located in CA1 stratum radiatum, to record the fEPSPs while stimulating presynaptic Schaffer collateral fibers. Field EPSPs slope was used to assess synaptic strength (Sánchez-Puelles et al, Cereb Cortex. 2020;30(2):505-524). For each slice, an input-output response curve was recorded to compare basal synaptic transmission (BST) at different conditions. The response curve was obtained by varying the intensity of single-pulse stimulation from 10 to 250 µA and averaging 5 responses per intensity. This curve was also used to set the baseline fEPSP at ≈30% of maximal slope. Following the baseline period (10-20 minutes of stable responses), LTP was induced through a three theta-burst stimulation (TBS) protocol (each burst consisting of 4 pulses at 100 Hz repeated with 200 ms inter-burst intervals) (Kimura and Ohno, Neurobiol Dis. 2009;33:229-35), and the responses were recorded for 1 h. To monitor the paired-pulse ratio (PPR), 2 pulses were separated by increasing interstimuli intervals (50, 100, 200, and 400 ms) (Sánchez-Puelles et al, Cereb Cortex. 2020;30(2):505-524). All electrophysiological data were collected with pCLAMP software (Molecular Devices) and analyzed blindly.

### Hippocampal extracts

Extracts from hippocampus were obtained in cold extraction buffer (20 mM Tris-HCl pH 6.8, 10 mM β-mercaptoethanol, 1 mM EDTA, 1% Triton X-100, 1% SDS) including Complete Mini, EDTA-free, protease inhibitor cocktail (Roche, Ref. 11836170001) (one hippocampus in 500 µl extraction buffer). Extracts were centrifuged for 10 min at 14,000 × g (at 4 °C), and supernatants were then boiled for 5 min in either tricine loading buffer (50 mM Tris-HCl pH 8, 12% Glicerol, 4% SDS, 0.001% Coomasie blue , 2% β-mercaptoethanol), for Aβ analysis, or Laemli buffer (62.5 mM Tris-HCl pH 6.8, 2% SDS, 10% Glicerol, 2% β-mercaptoethanol, 0.005% Bromphenol blue), for E2F4 quantification.

### Western blot

Extracts for E2F4 and actin analysis were fractionated in 4-20% TGX- precast protein gels (Bio-Rad, Ref. 4561095), and then transferred to 0.45 µm Immobilon-FL transfer membranes (Millipore, Ref. IPFL00010). For the analysis of Aβ levels, hippocampal extracts were fractionated on 16.5% Tris-tricine acrylamide gels (Bio-Rad, Ref. 4563063) and transferred to 0.2 µm Low-Fluorescence PVDF transfer membranes (Thermo Scientific, Ref. 22860). The membranes were incubated for 1 h at RT with Oddyssey Blocking Buffer (OBB) (Intercept^{®} Blocking Buffer (LI-COR, Ref. 927-60001) and then incubated ON at 4 °C with the primary antibodies at the appropriate dilution in OBB containing 0.1% Tween 20 (Sigma-Aldrich, ref. P1379) (OBB-T). After washing the membranes with OBB-T, they were incubated for 1 h at RT with the secondary antibodies in OBB-T. Finally, the membranes were washed again with OBB-T, and the protein bands were visualized using the Odyssey CLx Infrared Imaging System (Li-COR). Finally, the ImageJ software (Fiji) was used for the quantification of the bands.

### Behavioral Assays

Animals were handled for 5 min in the experimental room on each of the 3 days before beginning the experiments. Experimental mice were transferred to the behavioral testing room 30 min prior to beginning the first trial to habituate them to the room. To avoid the influence of circadian fluctuations, the behavioral tests were always performed in the morning. All behavioral parameters were scored blindly. Light levels were controlled in all the behavioral experiments. All apparatuses were cleaned with 0.1% Acetic Acid between mice to remove odors.

### Open Field

The Open Field test (OF) is the most common behavioral test to analyze the general motor function and the degree of basic activity of rodents, evaluating the changes in exploratory behavior. To study the spontaneous locomotor activity a VersaMax Legacy Open Field activity box (Omnitech Electronics) was used. Mice were allowed to move freely around the open field. The free movement of mice was recorded for 5 min. The distance traveled during the 5 min trial was measured.

### Elevated Plus Maze

The elevated plus maze (EPM) is used to measure anxiety-like behavior in laboratory animals. The test is based on the natural aversion of mice to open and elevated areas, as well as their natural spontaneous exploratory behavior in novel environments. The EPM was performed as previously described (Sánchez-Puelles et al, Cereb Cortex. 2020;30(2):505-524). Briefly, animals were placed in an elevated methacrylate maze (Cibertec) consisting of a plus-shaped apparatus with two open and two closed arms of equal length and width (30 x 6 cm) and a connecting central platform (6 x 6 cm), elevated 77 cm above the ground. The test consists of a single 5-minute trial for each animal, with the mouse placed in the center of the maze, facing an open arm and away from the experimenter. During each trial, the mouse was free to move along the apparatus and its movements were recorded using Tracking Interface software and analyzed using Video Maze software (Cibertec). The time spent in the open arms reflects the state of anxiety, with higher times indicating that the animals are less anxious.

### Novel Object Location (NOL)

Hippocampal-dependent memory was tested using the NOL task. This assay was chosen because it is not intrinsically stressful, a factor that crucially affects the cognitive performance of mice in other spatial learning paradigms (e.g., the Morris water maze) (Sánchez-Puelles et al, Cereb Cortex. 2020;30(2):505-524). The NOL task consisted of 3 phases: habituation phase, sample phase, and test phase. Mice were first individually habituated for 30 min to an empty open field white box [40×30×15(height) cm] during three consecutive days prior to the test. The box presented different patterns in each wall (triangles, lines, circles and stars) as spatial cues. The sample phase (object exposure) consisted of placing the mouse into the box that contained two identical objects during 10 min. Animals were free to explore the box and objects during this time. Once the sample phase was finished the mouse was removed from the test box and, after a 60 min delay (retention period), the mouse was placed back into the test box to perform the memory evaluation test. The test consisted of switching the location of one of the objects (Novel Object Location trial) and let the animal to freely explore for 5 minutes (Fig. 3c). Using the cumulative time exploring both objects in the choice session the percentage of total investigation was calculated for each object, dividing the time spent exploring the displaced, for novel location (%NL), or non-displaced, familiar location (%FL), object by the time spent exploring both objects. A %NL similar to 50 would correspond to equal exploration of both objects. Therefore, a %NL above 50 means that the animal has learnt the position of the objects in the sample phase and recognize the displaced one in the choice phase, thus indicating a normal/good hippocampal-dependent learning. Subjects were excluded from the analysis if they failed to explore both objects for a total of at least 10 s during the test sessions. Animal tracking and score was done using Ethovision XT 11.5 Software (Noldus) and Any-maze (Stoelting). Videos were recorded the day of the experiment and analyzed later blindly. Exploration is analyzed manually with the key manager and is identified when the animal is faced to the object and the nose is closed to it.

### Contextual Fear Conditioning (CFC)

CFC is a form of associative learning depending on hippocampus in which a neutral stimulus (a given spatial context, i.e. contextual stimulus) is associated with an aversive event (an electric shock, i.e. unconditioned stimulus). This test consists in two day sessions (Fig. 3h): training (conditioning session) and test (memory recall session) (Stefanelli et al, Neuron. 2016;89(5):1074-85). During the conditioning session, each mouse was individually placed in a methacrylate cage (inner dimensions 25x25x35(height) cm) containing a metallic grid floor and walls covered with different patterns (black and white lines, squares and smooth gray) to produce contextual cues, which was inserted into a sound-attenuating chamber. Mice were allowed to explore for 3 min and then, three mild electric shocks (0.5 mA, 2 s) were delivered through the stainless-steel floor grid with 30 s interval. Mice stayed 1 min additionally before being returned to the home cage (total conditioning time: 5 min). The memory recall session was performed 24 hours later. Mice were placed back in the cage described above (conditioning context) for 5 minutes, during which no footshock was delivered (Fig. 3h). Mice exhibited a range of conditioned fear responses, including freezing, defined as behavioral immobility except for movement needed for respiration (Fanselow MS, Ann N Y Acad Sci. 2003 Apr;985:125-34). Freezing was automatically scored and monitored by video recordings, which were digitized using Any-Maze Video Tracking System version 6.05 (Stoelting Co). Raw data, including freezing total time, was exported from Any-Maze. Freezing total time was divided by total time session to obtain the percentage of time freezing, which indicates the level of memory recall of mice. To evaluate if there are any differences in pain sensitivity between groups, what could affect the results of this experiment, pain threshold evaluation was done after the CFC test. In the conditioning chamber, after 120s of resting time, the electric current was gradually increased at a rate of 0.05 mA every 20 s until the animal showed the first signs of pain (jumping and vocalization). At this moment, the current was immediately switched off and the animal was back to its home cage after 30s of resting. The corresponding current was scored as a measure of the animal's pain threshold. Both assays, CFC and pain sensitivity threshold, were recorded using a camera fixed above the apparatus and connected to a computer.

### Statistical analyses

Quantitative data are represented as the mean ± standard error of the mean (s.e.m.). Statistical tests were carried out using GraphPad Prism version 7.00 for Windows (GraphPad Software; www.graphpad.com). Before performing any statistics, normality was evaluated. The data that passed normality Shapiro-Wilk test was analyzed using parametric tests. When two groups were analyzed, statistical differences were obtained between using two-tailed Student's t test, meanwhile when 3 or more groups are analyzed, one-way ANOVA analysis was performed followed by Tukey's or Dunnett's post hoc test. Two-way ANOVA analysis followed by Bonferroni post hoc test was also performed for BST experiments. The data that did not pass normality test was analyzed through nonparametric test, being the Mann-Whitney test used to compare two groups or Krustal-Wallis with Dunn's multiple comparisons post-test for 3 or more groups. Outliers, as evidenced by the Grubbs' test, also called the ESD method (extreme studentized deviate), were eliminated from the analysis. The details of the statistical test employed for each experiment appear in the figure legends.

### Results

### hE2F4DN localizes to the cytoplasm, dendrites, dendritic spines, and axon in cultured hippocampal neurons

To demonstrate the potential use of hE2F4DN as a therapeutic protein against Alzheimer's disease (AD), we first evaluated its subcellular localization in hippocampal primary culture neurons. To this end, we expressed myc-tagged, human E2F4DN (hE2F4DNmyc) in these cells under the control of the neuron-specific, *hSyn1* promoter using an adenoviral vector (Ad5.hE2F4DNmyc) (Fig. 1a). hE2F4DNmyc was mostly observed in the cytoplasm of neurons (Fig. 1b, d, e, f, g), which fits with the presence of two nuclear export signals in the E2F4 sequence. This result is also consistent with previous studies establishing non-canonical functions for E2F4 as a regulator of cell and tissue homeostasis, which is required for neuronal survival under cell stress conditions. Since hE2F4DN cannot be phosphorylated by the stress kinase p38^{MAPK}, likely maintaining the homeostatic role of the native E2F4 protein, we hypothesized that the expression of hE2F4DN could prevent AD-associated neuronal loss.

E2F4 can also interact with synaptic regulators. Consistent with this finding, hE2F4DNmyc was found in dendrites, dendritic spines (Fig. 1b, c, respectively) and axons (Fig. 1d, e), colocalizing with the synaptic marker synapsin (Fig. 1f, g). This is reminiscent of the synaptic localization of E2F1, another member of the E2F family that increases its expression in the brain with age, and triggers aging-associated cognitive loss. Taking together, these results indicate that E2F4 may be involved in synaptic function, likely as a modulator of synaptic plasticity in AD.

### Neuronal expression of hE2F4DN does not affect basal membrane properties

Since E2F4 can interact with synaptic regulators, hE2F4DN could potentially alter the basal electrophysiological properties of neurons, thus preventing its use as a therapeutic agent. To rule out this possibility, we evaluated the electrical membrane properties of cultured hippocampal neurons expressing the hE2F4DNmyc protein. To visualize neurons that were transduced (Fig. 2b, d, l), we used the adenoviral vector Ad5.hSyn1.EGFP to drive the expression of EGFP (EGFP) (Fig. 2a), as control condition, or the Ad5.hSyn1.hE2F4DNmyc-IRES-EGFP (hE2F4DN) (Fig. 2a) vector to co-express hE2F4DNmyc together with EGFP (Fig. 2d, e) specifically in neurons and with high efficiency (Fig. 2b, c). The internal ribosomal entry site (IRES) construct allows the expression of hE2F4DNmyc independently of the EGFP reporter. Then, we performed whole-cell patch clamp in neurons showing green fluorescence in both control and hE2F4DNmyc conditions (Fig. 2d, l). This analysis demonstrated that hE2F4DNmyc does not alter the membrane properties, as no significant changes were found in resting membrane potential (Fig. 2f), holding current (Fig. 2g), membrane resistance (Fig. 2h), and capacitance (Fig. 2i). The excitability of neurons was also tested by analyzing action potentials in both conditions. This analysis indicated that there are no significant changes in the number of events (Fig. 2j) or in the action potential firing threshold (Fig. 2k) when hippocampal neurons express hE2F4DNmyc. To analyze the spontaneous activity of hippocampal neurons in the presence of the therapeutic protein, both the frequency of spontaneous responses (sEPSCs,) and their amplitude were measured. As result, hE2F4DNmyc expression did not alter the spontaneous excitatory synaptic transmission compared to EGFP-expressing neurons (Fig. 2n and o, respectively).

These results demonstrate that hE2F4DNmyc expression does not affect neuronal membrane properties, neuron excitability or spontaneous synaptic responses, indicating that the use of hE2F4DN as a therapeutic protein is safe regarding neuronal physiology.

### In vivo administration of AAV.hE2F4DN prevents hippocampal-dependent memory loss in h5xFAD mice

We have previously shown that an hE2F4DN-based gene therapy using the AAV-PHP.B vector, known to efficiently cross the blood-brain barrier in C57BL6/J mice. When coupled to the human *hSyn1* promoter this vector was able to prevent cognitive loss in h5xFAD, as evaluated by Y-maze and Morris water maze, without triggering side effects. To confirm its therapeutic potential for AD, hE2F4DN was administered *in vivo* through another gene therapeutic approach, using the AAV-AS vector (Fig. 3a), which has previously been shown to cross the blood-brain barrier, driven by the *hSyn1* promoter.

The AAV-AS vector achieves a widespread neuronal transduction profile with a lower dose (5 × 10¹¹ vg/mouse) than other AAV capsids in adult mice. To confirm this observation, the AAV.hE2F4DNmyc vector was intravenously administered at two different doses, 3.12 × 10¹⁰ and 6.25 × 10¹⁰ vg/g body weight, in WT mice of 6 weeks of age and around 16 g of body weight to reach 5 × 10¹¹ and 1 × 10¹² vg/mouse, respectively (Fig. 8a). One month later, the expression of hE2F4DNmyc was analyzed by qRT-PCR in these mice. We observed that the mRNA expression levels rose linearly with increasing doses, resulting in a statistically significant correlation (Fig. 8b). For this characterization we used an antibody recognizing the myc tag, to specifically detect the expression of hE2F4DNmyc driven by the AAV.hE2F4DNmyc vector by immunohistochemistry. This analysis showed that around 60 % of neurons were positive for the myc tag in the cerebral cortex regardless of the dose (Fig. 8d, e). Therefore, we decided to use the lower dose, as in the study by Choudhury SR et al. (Mol Ther. 2016, 24(4):726-35), for further studies with this AAV vector.

We then confirmed that hE2F4DNmyc expression was maintained over time by quantifying its mRNA levels in animals administered with the vector at 6 weeks and sacrificed at 7 months (Fig. 8c). We also tested the possible toxicity of this vector by checking the leakage of the hSyn1 promoter in the liver as compared to the leakage of hE2F4DNmyc expression in this tissue observed with the AAV-PHP.B.hSyn1.hE2F4.WPRE3SL vector. According to its decreased tropism to liver, we observed that the administration of the AAV.hE2F4DNmyc at 3.12 × 10¹⁰ vg/g resulted in 1.48 ± 0.65 hE2F4-myc-positive cells per 10⁵ hepatocytes (n=2) one month after treatment. This number was reduced one order of magnitude when compared to that observed one month after AAV-PHP.B.hSyn1.hE2F4.WPRE3SL administration at this same titer (14.34 ± 4.89 myc-positive cells per 10⁵ hepatocytes;). Therefore, we concluded that the AAV-AS vector would be safer than the AAV-PHP.B vector for gene therapy delivery.

To test the efficacy of hE2F4DN, we examined whether the injection of AAVhE2F4DN could rescue hippocampal-dependent cognitive deficits in h5xFAD mice. To this aim, the hE2F4DN-expressing AAV-AS vector was intravenous injected at 3.12 × 10¹⁰ vg/g in 6-weeks-old WT and h5xFAD mice. At 5-6 months of age these mice were tested on two hippocampal-dependent cognitive tasks, NOL and CFC (Fig. 3c).

In the NOL test (Fig. 3c), both untreated WT mice and WT mice injected with the therapeutic vector spent significantly more time exploring the displaced object (Fig. 3f), thus reflecting that learning has been adequate in control mice and that hE2F4DN expression does not affect the formation of hippocampal-dependent memory. In contrast, non-treated h5xFAD mice showed a significant impairment in the NOL task, as both objects were equally explored (Fig. 3f). This indicates that h5xFAD mice do not distinguish between the displaced and the fixed object, reflecting impaired memory. Notably, although the administration of the therapeutic vector did not affect the performance of WT mice, the NOL test showed that administration of the AAV.hE2F4DN vector rescued the preference for novel objects, displaying a percentage of exploring time of the novel object significantly higher than familiar object (Fig. 3f). This result indicates that the neuronal expression of hE2F4DN prevents memory loss, as memory was intact at the time of injection (Fig. 3d). As a control, mice from the indicated experimental groups spent similar time exploring both objects during the sample phase (Fig. 9g,k). Moreover, hE2F4DN treatment did not significantly alter exploratory activity during the test phase, as the total time exploring the objects was similar between treated and not treated mice, although a non-statistically significant tendency to increased exploration was observed for the h5xFAD mice at both ages (Fig. 9h, l).

According with the results obtained by NOL test, we did not observe changes in the CFC paradigm at 5-6 months when WT mice treated with the therapeutic vector at 6 weeks of age were compared to untreated WT mice (Fig. 3i). Nevertheless, when h5xFAD mice were exposed to the CFC test, we observed that the administration of the therapeutic vector at 6 weeks of age resulted in the recovery of hippocampal-dependent memory (Fig. 3i). As a control, there was no significant changes in basal freezing behavior (Fig. 9m) or pain threshold (Fig. 9n) in animals of either group. Overall, we can conclude that neuronal hE2F4DN expression prevents memory loss in h5xFAD mice.

We also conducted other behavioral tests to evaluate the effect of the therapeutic vector on locomotor and anxious behavior (Fig. 9a-f). Locomotor activity was assessed through the Open Field test. h5xFAD mice display altered exploratory behavior with hyperlocomotive phenotype, beginning at 6 weeks of age (Fig. 9a). The open field test performed at 5-6 months of age showed no significant differences in the exploration between treated mice and their controls (Fig. 9c), although a non-significant increase in distance travelled was observed in not injected h5xFAD animals compared to the other groups. Anxious behavior was evaluated through the Elevated-Plus Maze test. This test resulted in no significant differences between WT and h5xFAD mice at 6 weeks of age (Fig. 9d). Notably, h5xFAD mice showed a statistically significant decrease in anxiety when compared to WT mice at 13 weeks of age (Fig. 9e), an effect that was maintained at 5-6 months of age (Fig. 9f). Nevertheless, we did not detect any difference between treated and not treated animals, what suggest that although the h5xFAD genotype is associated with disturbances on anxiety-related behavior, this is not modified by hE2F4DN expression.

### In vivo administration of AAV.hE2F4DN rescues hippocampal-dependent memory in h5xFAD mice

Since we were interested in a curative therapy for AD, we decided to apply the therapeutic vector in older mice, when the disease has already been developed. To this end, we focused on homozygous 13-week-old h5xFAD mice, an age at which these mice already display phenotypes associated with the disease. Most interestingly, we found that at this age hippocampus-dependent memory impairment is already detectable, as observed in the NOL paradigm (Fig. 3e). No differences were found in neither the exploration of the objects in the sample phase nor in total time exploring both objects in the test (Fig. 9i, j). NOL evaluation at 5-6 months of age in h5XFAD mice injected at 13 weeks of age with the therapeutic vector resulted in a significant memory improvement, compared to untreated h5xFAD mice (Fig. 3f). Furthermore, a significant increase in freezing behavior could be observed in 13-weeks-injected h5xFAD mice subjected to the CFC paradigm, when compared to non-treated mice, reaching similar values to those obtained in animals injected at 6 weeks of age (Fig. 3i).

Taken together, these data suggest that AAV.hE2F4DN improved the performance of h5XFAD mice in hippocampal-dependent learning/memory tasks, not only preventing but also recovering memory once it is already impaired. Looking at this promising data, the next step was to try to elucidate the mechanisms by which hE2F4DN is retrieving the memory.

### In vivo administration of AAV.hE2F4DN reduces Aβ pathology in h5xFAD mice

There is increasing evidence that Aβ accumulations represent an early step in the course of AD and that it plays a crucial role in synapse malfunction, leading to cognitive deficits, and posterior neuron loss in Alzheimer patients and AD transgenic mouse models. Therefore, we first evaluated the capacity of AAV.hE2F4DN to prevent Aβ production and deposition. A previous study using AAV.PHP.B vector to express hE2F4DN in neurons demonstrated that this treatment resulted in reduced Aβ accumulation in h5xFAD mice. We therefore studied whether AAV.hE2F4DN could also have disease-modifying properties, by evaluating its effects on Aβ pathology in h5xFAD mice.

5xFAD mice are known to harbor robust Aβ pathology, with the onset of extracellular plaque deposition at around 2 months of age, preceded by detectable intraneuronal Aβ accumulation as early as 6 weeks of age. This phenotype is aggravated in h5xFAD mice since a significant increase in Aβ peptide production and Aβ plaque load can be observed in different brain regions of these mice, compared to heterozygous 5xFAD mice. To evaluate the effect of the AAV.hE2F4DN gene therapy on the Aβ phenotype of h5xFAD mice, we measured the levels of soluble Aβ and plaques load in control and treated h5xFAD mice (Fig. 4). On the one hand, this study resulted in a significant increase of soluble Aβ monomers and oligomers (oAβ) when hippocampal fractions from h5xFAD mice of 6 months of age were compared by western blot to those from WT mice, an effect that was reduced by AAV.hE2F4DN independently on the age (6 weeks vs 13 weeks) in which the therapeutic vector was administered (Fig. 4 a, b, d). This finding was further substantiated since a significant negative correlation was observed between the amount of E2F4 protein and the quantity of both oAβ (Fig. 4c) and Aβ (Fig. 4e). On the other hand, the average Aβ plaque area observed at 6 months in the hippocampus of h5xFAD mice (Fig. 4f), was significantly reduced after neuronal expression of hE2F4DN, independently of the stage (6 weeks vs 13 weeks) in which the AAV.hE2F4DN vector was administered (Fig. 4f, g). In contrast, no changes were observed in plaque density (Fig. 4h). Taken together, these data confirms that AAV.hE2F4DN treatment at 6 weeks reduces Aβ production, thus reducing insoluble Aβ burden. Importantly, this same conclusion was obtained when the therapeutic vector was administered at 13 weeks, when cognitive loss is already evident. Nevertheless, this was a partial effect since the reduction of both Aβ production and accumulation was just around 30-40%. Therefore, other effects of hE2F4DN are likely underlying its therapeutic capacity.

### In vivo administration of AAV.hE2F4DN prevents synaptic plasticity loss in h5xFAD mice

Synaptic degeneration and neuronal loss are known to contribute to memory impairment in AD, paralleling the progression of cognitive decline. Therefore, we first explored whether cognitive recovery observed in h5xFAD mice correlated with synaptic function improvement. To this aim, synaptic plasticity was evaluated in hippocampal slices from control and h5xFAD mice *ex vivo.* We reasoned that, if AD-induced cognitive impairment results from a general synaptic impairment, including LTP, cognitive decay might be prevented by dampening this deficit in synaptic plasticity, would support the therapeutic effect of hE2F4DN. We therefore evaluated synaptic function by electrophysiological field recordings of excitatory postsynaptic potentials (fEPSPs) of CA3-CA1 synapses using acute slices from 6-month-old control and h5xFAD mice. Animals were intravenously injected at 6 weeks of age with the AAV.hE2F4DN vector or left untreated (Fig. 3b). At 6 weeks we did not detect differences between h5xFAD and control mice either in basal synaptic transmission (Fig. 5a), paired-pulse facilitation (Fig. 10a) or long-term potentiation (LTP) (Fig. 5d, e). In contrast, a 79% decrease in the maximum slope of synaptic responses (input-output curve) was observed in the slices from 6-7 months old h5xFAD mice compared to WT slices (Fig. 5c). This decrease in BST was partially prevented in h5xFAD mice injected with the AAV.hE2F4DN vector at 6 weeks (Fig. 5c). Interestingly, the beneficial effect observed in hippocampal slices from hE2F4DN-treated h5xFAD mice, was not observed in slices from hE2F4DN-treated WT mice, which did not significantly differ from those from untreated WT mice (Fig. 5c). Presynaptic short-term plasticity was similar in h5xFAD and WT mice, and did not change by hE2F4DN expression under these conditions (Fig. 10c).

We next evaluated the long-term effects of AAV.hE2F4DN treatment at 6-7 months. As expected, LTP was impaired in the hippocampal slices of h5xFAD mice, and this effect was prevented by AAV.hE2F4DN administration at 6 weeks (Fig. 5 h, i), since the LTP levels in hE2F4DN-treated h5xFAD mice were similar to those observed in WT mice (Fig. 5i). As a control, we found that hE2F4DN-expressing slices from WT mice also exhibited significant LTP, at similar levels to those observed in hippocampal slices from non-injected control mice (Fig. 5h, i). Thus, hE2F4DN prevents AD-induced impairments by boosting both BST and LTP.

### In vivo administration of AAV.hE2F4DN rescues LTP loss in h5xFAD mice

We have demonstrated that 5hxFAD mice presents hippocampal-dependent memory impairment already at 13 weeks, a cognitive phenotype that was rescued by neuronal hE2F4DN expression (Fig. 3). Therefore, we decided to evaluate whether the studied parameters of synaptic plasticity (i.e. BST, PPF, and LTP) were also altered at this stage. The analysis revealed that the synaptic strength between CA3-CA1 synapses at 13 weeks was reduced (Fig. 5b) and LTP was impaired in h5xFAD mice (Fig. 5f, g). In contrast, no difference in presynaptic plasticity was found between h5xFAD and WT mice at 13 weeks of age (Fig. 10b).

Once demonstrated that synaptic plasticity was already impaired at 13 weeks in h5xFAD mice, we decided to administer the AAV.hE2F4DN vector at this age to evaluate the capacity of the therapy to recover the synaptic plasticity deficits, thus being a potential therapeutic candidate for AD. Mice were intravenously injected with the AAV.hE2F4DN vector at 13 weeks and field recordings were subsequently done at 6-7 months of age (Fig. 3b). This analysis indicated that BST is slightly but significantly increased (Fig. 5c). Nevertheless, h5xFADmice injected with the AAV.hE2F4DN vector at 13 weeks showed a significant recovery of LTP levels (Fig. 5h, i).

Taking together, these results suggest that hE2F4DN activity can rescue the synaptic pathology associated to AD, controlling the strength of synaptic plasticity and LTP expression, thus promoting the recovery of memory capabilities. Importantly, our *ex vivo* results confirm that hE2F4DN expression does not trigger side effects on the electrophysiological properties of WT neurons, as described above (Fig. 2).

### In vivo administration of AAV.hE2F4DN increases PSD-95 levels in h5xFAD mice

Correlating with cognitive decline, 5xFAD mice show a decrease in PSD-95, a scaffolding protein highly stable at the synapse. This protein is known to bind to and to stabilize NMDA receptors at the postsynaptic side, which is necessary for the induction of LTP. Therefore, the stabilization of PSD-95 might participate in the recovery of the LTP process found in h5xFAD mice treated with hE2F4DN.

To investigate whether neuronal hE2F4DN expression can regulate excitatory synaptic function by modulating the expression of PSD-95, we analyzed this marker in hippocampal extracts from 6-month-old mice. We confirmed that the levels of PSD-95 were decreased in h5xFAD compared with WT mice (Fig. 6c). Moreover, h5xFAD injected mice, at both 6 and 13 weeks of age, showed increased levels of PSD-95 compared to untreated h5xFAD animals (Fig. 6c), an increase that positively correlates with the hE2F4DN protein levels (Fig. 6d).

Taken together, these results indicate that the expression of hE2F4DN in neurons favors the upregulation of PSD-95, which may be contribute to the LTP rescue and the behavioral improvement observed in hE2F4DN treated h5xFAD mice compared with control ones.

### In vivo administration of AAV.hE2F4DN reduces neuronal death in h5xFAD mice

Previous analyses of the brain of 5xFAD mice demonstrated significant neuron loss at 9 months of age, an effect likely participating in the progression of the cognitive decline observed in these mice, as occurs in AD patients. Therefore, we decided to explore whether cognitive recovery observed in h5xFAD mice is associated with reduced neuronal death.

To assess the consequences of hE2F4DN expression on neuronal survival we used unbiased stereology to determine the number of neurons in the hippocampal CA1 pyramidal layer of control and h5xFAD mice. Coronal brain sections from 6-month-old h5xFAD and non-transgenic controls, untreated or treated with AAV.hE2F4DN at 6 weeks of age, were stained with cresyl violet to visualize neuronal soma (Fig. 7a, b). At 6 months, untreated h5xFAD mice showed a 45% loss of pyramidal neurons in the CA1 region of the hippocampus compared to WT mice (Fig. 7c), which correlated with a 40% decrease in CA1 volume (Fig. 7d). Remarkably, the observed reduction in the number of CA1 pyramidal neurons as well as in the volume of the hippocampal CA1 region was not observed in h5xFAD mice injected with the AAV.hE2F4DN vector, showing similar values to control mice (Fig. 7c, d). Therefore, hE2F4DN expression prevents the neuronal death associated to AD. In contrast, non-transgenic control mice injected with the therapeutic vector did not show changes in these parameters (Fig. 7c, d).

Strikingly, the administration of AAV.hE2F4DN at 13 weeks of age, when cognition is already affected in h5xFAD mice (Fig. 3e), was still able to rescue the neuronal death observed in hippocampal CA1 pyramidal neurons (Fig. 7c), resulting in the maintenance of the volume of this hippocampal structure (Fig.7d). Taking them together, these results demonstrate that hE2F4DN expression fully prevents neuronal loss in h5xFAD mice, without triggering side effects in control conditions. This effect likely participates in the recovery of memory formation observed in h5xFAD mice with neuronal expression of hE2F4DN.

### Example 2

### Materials and Methods

### Mice

For this study, 24 C57BL/6JOlaHsd female mice of 11 weeks of age were purchased from Envigo (Barcelona, Spain).

### AAV vectors

**Table 1. Detailed features of the two viral vectors and the formulation buffer employed in this study.**

| *Test item* | **AAV-AS.hSyn1.hE2F4 DN.WPRE3SL (AAV.hE2F4DN)** | **AAV9.hSyn1.hE2F4DN.W PRE3SL (AAV9.hE2F4DN)** | **AAV9.hE2F4DN formulation buffer** |
|---|---|---|---|
| | | | Water for Injection |
| | | | 8.1 mM Na2HPO4.7H20 |
| | | | 1.5 mM K2HPO4 |
| | | | 2.7 mM KCl |
| *Concentration* | 5×10¹³ vg/mL | 2.9×10¹³ vg/mL | 137 mM NaCl |
| | | | 0.5 mM MgCl2 |
| | | | 0.9 mM CaCl2 |
| | | | 4.4 mM Dextrose |
| | | | 0.001% Poloxamer 188 |
| *Active ingredient* | AAV9 vector (rAAV of serotype 9 encoding the transgene hE2F4DN under the control of the human Synapsin1 promoter) | AAV9 vector (rAAV of serotype 9 encoding the transgene hE2F4DN under the control of the human Synapsin1 promoter) | N/A |

### AAV intracisternal administration

Intracisternal administration of the AAV9.hE2F4DN vector was performed at 1 µl/min as described by Xavier et al. (J Vis Exp. 2018;(135):57378).

### RNA Extraction and cDNA Synthesis

Total RNA was extracted using QIAzol Reagent (Qiagen) and cleaned with the column based Rneasy mini kit (Qiagen, #74104) following manufacturer's instruction. Synthesis of cDNA was performed using SuperScript IV Reverse Transcriptase (ThermoFisher Scientifc) following the manufacturer's instructions.

### Genomic DNA extraction

Genomic DNA was extracted as described by Laird et al (Nucleic Acids Res. 1991;19(15):4293).

### qPCR

Genomic DNAs were amplified with specific primers for the viral genome using an SYBR Green kit (BioRad). Kcna3-specific primers were used for normalization.

### qRT-PCR

cDNAs were amplified with specific primers for the transgene construct using an SYBR Green kit (BioRad). Rps18-specific primers were used for normalization.

### Experimental protocol

Six experimental groups of 11-week-old WT female mice (n=4 per group) were used in the present study.
- Control: non injected.
- AAV9.hE2F4DN (Dose 1): intracisternal administration of 1×10¹⁰ vg in 18 µl formulation buffer (2.38×10¹³ vg/kg brain).
- AAV9.hE2F4DN (Dose 2): intracisternal administration of 5×10¹⁰ vg in 18 µl formulation buffer (1.19×10¹⁴ vg/kg brain).
- AAV9.hE2F4DN (Dose 3): intracisternal administration of 1×10¹¹ vg in 18 µl formulation buffer (2.38×10¹⁴ vg/kg brain).
- AAV9.hE2F4DN (Dose 4): intracisternal administration of 5×10¹¹ vg in 18 µl formulation buffer (1.19×10¹⁵ vg/kg brain).
- AAV.hE2F4DN: intravenous administration of 6.56·10¹¹ vg (3.125 × 10¹³ vg/kg).

This comparison can provide an idea of the therapeutic dose of AAV9.hE2F4DN. The volume infused through the cisterna magna was 18 µL per mouse, with a flow rate of 1 µL/min. At this flow rate no side effects have been observed in mice.

Fourteen days after administration of the vectors, mice were sacrificed and the cerebral hemicortices were dissected as described in TET-SOP-LAB-010. Genomic DNA was isolated from the left hemisphere and total RNA from the right hemisphere, and the presence of the viral vector as well as the expression of the *E2f4dn* transgene were studied by qPCR.

### Results

The hE2f4DN/Rps18 ratios were obtained for each experimental point are shown in Fig. 11.

By interpolation, we have determined the intracisternal dose of AAV9.hE2F4DN that achieves a hE2f4DN/Rps18 ratio in the cerebral cortex similar to that of intravenous administration with the AAV.hE2F4DN vector at its effective dose (3.125 × 10¹³ vg/kg): **2.04 × 10¹¹ vg/brain** (in mice, the brain weights 0.42 g) (Figure 12).

### Conclusion

Comparative analysis between intravenous administration of the AAV.hE2F4DN vector at 3.125 × 10¹³ vg/kg and different doses of the AAV9.hE2F4DN vector administered intracisternally (1 × 10¹⁰, 5 × 10¹⁰, 1 × 10¹¹ and 5 × 10¹¹ vg/brain) in wild-type mice has allowed the determination of the therapeutic dose of the latter vector.

Results indicated that the dose considered effective is 2.04 × 10¹¹ total vg per mouse. This dose is equivalent to 4.86 × 10¹⁴ vg/kg of brain since a mouse brain weights 0.42 g.

The intracisternal AAV9.hE2F4DN dose of 4.86E+14 vg/kg brain could be directly extrapolated to humans.

## Claims

1. A polynucleotide encoding a mutant form of the human E2F4 transcription factor in which positions 248 and/or 250 are mutated so that they cannot be phosphorylated for use in the treatment of Alzheimer's Disease in a patient diagnosed as having Alzheimer's Disease and having mild, moderate or severe dementia.

2. The polynucleotide for use according to claim 1 wherein the positions 248 and/or 250 contain alanine residues.

3. The polynucleotide for use according to claim 1 or 2 wherein the viral vector is an adeno-associated virus vector.

4. The polynucleotide for use according to claim 3, wherein the adeno-associated virus vector is AAV9.

5. The polynucleotide for use according to claim 3 or 4, wherein the adeno-associated virus vector is a self-complementary AAV.

6. The polynucleotide for use according to any one of claims 3 to 5, wherein the polynucleotide within the vector is operably controlled by a neuron-specific promoter, wherein the neuron-specific promoter is human Synapsin 1 promoter.

7. The polynucleotide for use according to any one of claims 1 to 6, wherein the polynucleotide is administered into the cisterna magna or by intravenous route.

8. A pharmaceutical composition comprising an adeno-associated virus serotype 9 (AAV9) vector which comprises a polynucleotide that encodes a mutant form of the human E2F4 transcription factor in which positions 248 and/or 250 are mutated so that they cannot be phosphorylated for use in the prevention and/or treatment of Alzheimer's disease wherein the pharmaceutical composition is administered to achieve between 0.5 × 10¹⁴ and 6 × 10¹⁴ viral genomes (vg) of the adeno-associated virus serotype 9 (AAV9) vector per kg of brain.

9. The pharmaceutical composition for use according to claim 8 wherein pharmaceutical composition is administered to achieve between 1.21 × 10¹⁴ and 4.86 × 10¹⁴ vg/kg of brain.

10. The pharmaceutical composition for use according to any one of claims 8 or 9, wherein the content of viable viral genomes within the pharmaceutical composition is of 35-50% with respect to the total number of viral genomes.

11. The pharmaceutical composition for use according to claim 10, wherein the content of viable viral genomes within the pharmaceutical composition is of about 40 % of the total number of viral genomes.

12. The pharmaceutical composition for use according to any of claims 8 to 11 wherein the pharmaceutical composition is formulated using dextrose solution in PBS and wherein the dextrose is present at a concentration that provides an osmolarity to the pharmaceutical composition similar to that of the human CSF.

13. The pharmaceutical composition for use according to any of claims 8 to 12 wherein the composition is administered intracisternally.

14. The pharmaceutical composition for use according to any of claims 8 to 14 wherein the composition is a single dose composition

15. The pharmaceutical composition for use according to any of claims 8 to 15 wherein the composition is administered only once within the lifetime of the patient
